# EUROPEAN PATENT SPECIFICATION

(11) **EP 3 953 706 B1**
(45) Date of publication and mention of the grant of the patent: **01.07.2026**
(21) Application number: 20722904.8
(22) Date of filing: 09.04.2020
(51) Int. Cl.: G01N 33/487, A61B 5/145

(54) **SYSTEM AND METHOD TO LOCATE GLUCOSE SOURCES OR DIABETES TESTING SUPPLIES**
SYSTEM UND VERFAHREN ZUR LOKALISIERUNG VON GLUCOSEQUELLEN ODER DIABETESTESTMATERIAL
SYSTÈME ET PROCÉDÉ POUR LOCALISER DES SOURCES DE GLUCOSE OU DES FOURNITURES DE TEST DE DIABÈTE

(30) Priority: 11.04.2019 US 201916382113
(43) Date of publication of application: 16.02.2022
(73) Proprietor: F. Hoffmann-La Roche AG, 4070 Basel (CH); Roche Diabetes Care GmbH, 68305 Mannheim (DE)
(72) Inventor: MEARS, Mark Gilmore, Indianapolis, Indiana 46250 (US)
(74) Representative: Altmann Stößel Dick Patentanwälte PartG mbB
(86) International application number: PCT/US2020/027500
(87) International publication number: WO 2020/210514

(56) References cited:
- WO-A2-02/05702
- WO-A2-2016/179559
- US-A1- 2011 124 996
- US-A1- 2012 083 669
- US-A1- 2014 012 117
- EILAND LESLIE ET AL: "App-Based Insulin Calculators: Current and Future State", CURRENT DIABETES REPORTS, CURRENT SCIENCE, PHILADELPHIA, VA, US, vol. 18, no. 11, 4 October 2018 (2018-10-04), pages 1 - 13, XP036620114, ISSN: 1534-4827, [retrieved on 20181004], DOI: 10.1007/S11892-018-1097-Y

## Description

### CROSS REFERENCE TO RELATED APPLICATIONS

This application claims benefit of Non-Provisional U.S. Patent Application No. 16/382,113, filed April 11, 2019.

### TECHNICAL FIELD

This disclosure relates to a method for the management of diabetes mellitus generally and, more specifically, to a method that locate glucose sources

### BACKGROUND

Individuals suffering from diabetes may have difficulty producing sufficient insulin in their bodies to process the glucose or sugar in their blood *(e.g.,* Type 1 diabetes) or responding to certain levels of insulin (e.g., Type 2 diabetes). Hyperglycemia is a diabetic condition in which one's blood glucose rises to excessive or dangerous levels and causes serious health problems. A healthy diet and limiting carbs can assist in glucose control for individuals having Type 2 diabetes. Medication is also used to help control blood glucose levels for individuals with diabetes.

Diabetics may encounter occasions in which there is a mismatch between their medication regimen, food intake, or exercise routine that results in hypoglycemia, or excessively low blood sugar levels. For example, taking too much insulin medication or exercising harder than usual may result in too much glucose being processed from the blood. Non-diabetics also experience hypoglycemia in some cases. To treat hypoglycemia, individuals consume foods that are high in carbohydrates to increase their blood glucose levels. However, if the individuals are beginning to experience certain symptoms of hypoglycemia, such as confusion, disorientation, or lack of concentration, they may have difficulty locating sources of carbohydrates. The individual's inability to locate or consume carbohydrates may lead to more serious medical symptoms or complications, such as seizures, coma or death.

Current location services that are offered to users fail to provide proper assistance to individuals with diabetes or diabetic conditions. The current location services may cause an individual, who may be in an already confused or disoriented state, to have to filter through a lot of information to identify locations at which the individual may access sources of carbohydrates or medication to help treat the individual's condition. In fact, as location services become more advanced, location services that are offered to users continue to offer more and more information about an establishment at a given location. As treatment of a diabetic condition is often a time sensitive matter, users of current location services may not have the time, or the mental capacity, to search through the mass of information being provided. Additionally, the processing and network resources that are utilized for providing the mass amount of information location services continues to increase, which can also increase the amount of time and delay for an individual to receive helpful information.
US 2014/012117 A1 discloses a variety of capabilities of modern smartphones, and combines these capabilities with information from a continuous glucose monitor to provide diabetics and related people with more information than the continuous glucose monitor can provide by itself.

### SUMMARY

In some embodiments, a user device assists a user in locating sources of food items (e.g., carb-related food items) during a diabetic-related event, or when a triggering event is detected by the user device. In some embodiments, the user device is a mobile device, a glucose monitoring device, a diabetes medical device, a remote computing device *(e.g.,* a smart refrigerator), or any combination thereof. In some embodiments, the triggering event includes detection of an extreme diabetic event, such as hyperglycemia or hypoglycemia. In some embodiments, the triggering event includes a request for assistance by the user of the user device. The user device then obtains information regarding its location and information regarding food items that are within a predefined area or predefined distance of the user device.

For example, the information regarding the food items includes when the items were purchased by the user, available sources of the food items, locations of the sources of food items, or nutritional information associated with the food items (e.g., their glycemic index). The user device displays the information regarding the food items, that are within a predefined area or predefined distance of the user device. The user device displays the information in a predefined order or list. For example, the user device displays a list of food items in order of their glycemic index or purchase date. The user device displays a list of the sources of food items in order of travel time proximity to the user device or in order of their availability.

The user device obtains information regarding its location via global positioning system (GPS) signals, Wi-Fi signals, Bluetooth beacon signals, near-field communication (NFC) signals, or another RF communication signal. The location information includes a floor of a building on which the user device is located, a latitude and longitude of the user device, a street address, or a user-friendly location name.

The user device assists the user in locating carb-related food items within the home *(e.g.,* during an extreme diabetic event). For example, the user device assists the user in locating carb-related food items in the refrigerator or recently purchased carb-related food items. The user device displays food items in the refrigerator with a name, a photo, or a location in the refrigerator. The user device displays food items purchased within a predefined period of time with a name or a photo. The user device receives a universal product code (UPC) associated with one or more food items that indicates the glycemic index of the food items.

The user device receives images of food items, which are in a refrigerator. The user device compares the images of the food items with other (e.g., known) images of carb-related food items. The user device displays the images of the food items in the refrigerator in response to a threshold level of similar features being detected between the images of the food items in the refrigerator and the other images of carb-related food items. The user device receives one or more images of the carb-related food items in the refrigerator, the receipt of which indicates the detection of the carb-related food items by a camera of the refrigerator.

The user device assists the user in locating sources of carb-related food items outside the home (e.g., during an extreme diabetic event). The user device detects establishments that sell carb-related food items above a given nutritional value that are within a predefined distance or travel time.

In some embodiments, the user device filters establishments that sell carb-related food items based on certain criteria. For example, the user device receives hours of operation for the locations of the sources of food items outside the user's home and displays a subset of the locations of the sources of food items outside the home that are currently open based on the hours of operation. The user device receives an accepted form of payment at the locations of the sources of food items outside the home and displays a subset of the locations of the sources of food items outside the home that accept one or more predefined forms of payment. The user device receives an indication of the locations of the sources of food items outside the home that sell non-diet beverages and displays a subset of the locations of the sources of food items outside the home that sell the non-diet beverages.

The user device sorts establishments that sell carb-related food items based on certain criteria. The user device receives an indication of a user selection of a mode of transportation and display the locations of the sources of food items outside of the home in the order of travel time proximity based on the selected mode of travel. Locations of sources of food items are displayed with an establishment name, a latitude and longitude, an indoor identifier, an outdoor identifier, a user-friendly location name, a picture, an indication of public accessibility, or a map. In some embodiments, the locations of sources of food items includes a location of a vending machine.

The user device may receive an indication of a user selection of one of the locations of the sources of food items. The user device then provides the selected location of the source of food items to a map application on the user device for guiding the user to the selected location.

### BRIEF DESCRIPTION OF THE DRAWINGS

FIG. 1 is a perspective view of a representative environment for monitoring or treating a diabetic condition.
FIGs. 2A-2C illustrate a flow diagram of an exemplary process for assisting a user in locating sources of specific food, such as food that is high in carbohydrates.
FIGs. 3A and 3B illustrate a flow diagram of an non claimed process for assisting a user in locating sources of medical supplies or medical assistance.
FIGs. 4A-4E illustrate example graphical user interfaces (GUIs) on a display of a user device.
FIG. 5 is a block diagram of an example computing device.
FIG. 6 is a block diagram of an example blood glucose monitoring device.
FIG. 7 is a block diagram of an example blood glucose measuring (BGM) device.
FIG. 8 is a block diagram illustrating an example of an insulin pump.

### DETAILED DESCRIPTION

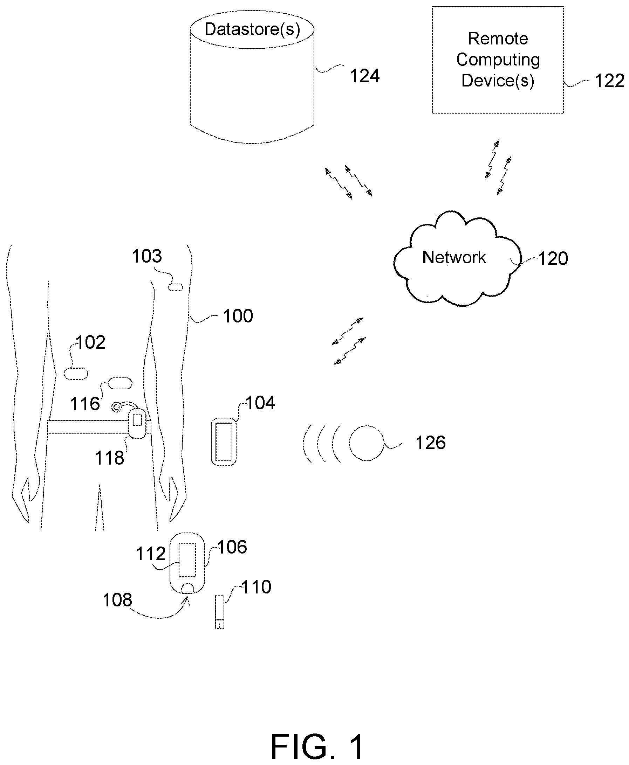

FIG. 1 is a perspective view of a representative environment for monitoring or treating a diabetic condition. As shown in FIG. 1, a user 100 with diabetes mellitus (referred to herein as "diabetes") may use one or more devices to help monitor or treat the user's diabetic condition. The diabetic condition includes a metabolic syndrome, pre-diabetes, type 1 diabetes, type 2 diabetes, or gestational diabetes. The user 100 may enter an extreme diabetic state, such as hypoglycemia or hyperglycemia, when the blood glucose level of the user 100 is below or above a threshold blood glucose level. The user 100 may use a blood glucose monitoring device to monitor blood glucose levels.

As used herein, the term "blood glucose monitoring device" refers to any device that detects and reports a level of glucose in the blood of the user, either through direct measurement of the blood or through an indirect detection process. A blood glucose level is also referred to as a blood sugar level. Examples of blood glucose monitoring devices include, but are not strictly limited to, continuous glucose monitoring devices, flash glucose monitoring devices, and blood glucose meters that provide a single measurement of blood glucose levels from a blood sample in a "spot" monitoring process. FIG. 1 depicts examples of blood glucose monitoring devices that are described in more detail below.

As used herein, the term "mobile device" refers to any mobile electronic device that is capable of moving with a user as the user changes locations. Example mobile devices include mobile phones, smartphones, wearable devices, tablets, laptops, notebook computers, personal digital assistants (PDAs), and any other mobile electronic device that is capable of moving with a user. Some embodiments of the mobile device incorporate the blood glucose monitor into an integrated device.

In some embodiments, the blood glucose monitoring device is a continuous glucose monitor (CGM) 102. The CGM 102 includes a subcutaneous sensor that is used to sense and monitor the amount of glucose in interstitial fluid of the user 100. The CGM 102 includes a transmitting device that is located directly over the sensor that wirelessly powers the data transfer from the sensor. The CGM 102 periodically communicates data indicating the blood glucose levels of the user 100 to an external device, such as a mobile device 104, for computing or storing the blood glucose levels of the user 100. In some embodiments, the mobile device 104 operates as a CGM controller device. Though the mobile device 104 is provided as an example of a device with which the CGM 102 communicates, the CGM 102 may communicate with other dedicated CGM controller devices for providing similar functionality that is described herein for the mobile device 104. The mobile device 104, or another CGM controller device, provides audible alerts, graphical user interfaces, or non-audible alerts in response to data indications or triggers identified in the monitored blood glucose data. The CGM 102 may include the sensor implanted in the user 100 and the transmitter, which may be applied to the body of the user 100. In one embodiment, the CGM 102 processes the blood glucose data for providing alerts. In another embodiment, the blood glucose data is processed at the mobile device 104, or other CGM controller device, and an alert indicator is communicated to the CGM 102.

In some embodiments, the blood glucose monitoring is performed by flash glucose monitoring (FGM). The FGM includes a subcutaneous sensor 103 that is used to sense and monitor the amount of glucose in interstitial fluid of the user 100. A separate reader device, such as the mobile device 102 or another reader device, receives the blood glucose information from the sensor when the device is within the RF range of the sensor 103. The sensor 103 transmits an instantaneous blood glucose level or a graphical trend of the blood glucose level to the reader device for display.

The user 100 may use a blood glucose meter (BGM) 106 as a blood glucose monitoring device to monitor blood glucose levels. The BGM 106 includes a port 108 that receives a blood glucose measurement strip 110. The user 100 may deposit a sample of blood on the blood glucose measurement strip 110. The BGM 106 analyzes the sample and measures the blood glucose level in the sample. The blood glucose level measured from the sample is displayed on a display 112 of the BGM 106 or communicated to an external device, such as the mobile device 104.

The blood glucose level measured by the BGM 106 or computed using data received from the CGM 102 is used to treat the diabetic condition of the user 100. For example, the user 100 may use an ambulatory non-durable insulin pump 116 or an ambulatory durable insulin pump 118 to treat the diabetic condition with insulin. The mobile device 104 determines an amount of insulin to be administered to the user 100 and the insulin pump 116, 118 receives instructions from the mobile device 104 to deliver a predetermined amount of insulin to the user 100. In some embodiments, the insulin pump 116, 118 receives other information from the mobile device 104, such as mealtime information or exercise information of the user 100. The insulin pump 116, 118 determines the amount of insulin to administer based on the received information from the mobile device 104. The insulin pump 116, 118 communicates information to the mobile device 104. The information communicated to the mobile device 104 includes an amount of insulin delivered to the user 100, corresponding times of delivery, or a pump status (e.g., battery status, insulin status, or another status of a portion of the pump).

The mobile device 104 communicates with the insulin pump 116, 118, the CGM 102, or the BGM 106 using wired or wireless communications. The mobile device 104, the CGM 102, a CGM controller, the BGM 106, or the insulin pump 116, 118 are collectively referred to as user devices. The mobile device 104 communicates with the other user devices using the same or different wireless protocols. For example, the mobile device 104 communicates with the other user devices using BLUETOOTH^{®}, near field communication (NFC), THREAD^{®}, WIFI^{®}, ZIGBEE^{®}, WI-MAX^{®}, a cellular communication protocol, a proprietary wireless communication protocol, or another radio frequency (RF) communication protocol.

In some embodiments, the mobile device 104 is a mobile phone, a tablet, a laptop, a wearable device, a personal digital assistant (PDA), or another mobile computing device. If the mobile device 104 is a wearable device, the wearable device may be an armband *(e.g.,* a smart watch, such as an APPLE^{®} watch, a FITBIT^{®} armband, or other device capable of being worn on the arm of the user 100), a ring, glasses *(e.g.,* GOOGLE^{®} GLASS^{™}), a headset *(e.g.,* BLUETOOTH^{®} headset), clothing (e.g., shirts, gloves, or another article of clothing), or another wearable device capable of being worn by the user 100. The mobile device 104 may include an electric motor for providing on-body vibration alerts and/or a speaker for providing audible alerts in response to data indications or triggers identified in the monitored blood glucose data. Though a mobile device is described herein, other computing devices may be similarly implemented to perform functions of the mobile device 104.

The mobile device 104 receives data and store data for assisting in monitoring or treating the diabetic condition. The mobile device 104 may receive input from the user 104 via a user interface being provided on a display. The mobile device 104 receives input via hard buttons or soft buttons provided on the display.

In some embodiments, the mobile device 104 is configured to determine the device's location. For example, the mobile device 104 is able to determine the geolocation (e.g., latitude and longitude) of the device using signals from a global positioning system (GPS) or triangulation via cellular communications. In some embodiments, the mobile device 104 determines a relative location using an RF beacon device 126. The RF beacon device 126 communicates a unique identifier via a short-range wireless communication, such as a BLUETOOTH^{®} low energy (BLE) beacon or an NFC beacon. The mobile device 104 receives the RF beacon and performs a lookup in a database (e.g., in information from the datastores 124) to determine a relative location associated with the unique identifier. For example, the mobile device 104 determines that the RF beacon indicates that the device is in a particular room in a home or building, on a certain floor in a building, close to a predefined object, or is within the RF range of a beacon associated with another object or location.

The mobile device 104 includes one or more sensors for detecting a relative position of the device or information about the user 100. The mobile device 104 detects a movement or a change in orientation. Based on the movement or change in orientation (or lack thereof) of the mobile device 104 over a period of time, the mobile device 104 detects that the user 100 is standing, sitting, or lying down. The mobile device 104 detects that the user 100 is exercising when the movement or the change in orientation is greater than a threshold for a period of time. The mobile device 104 detects the heartrate of the user 100 using a heartrate sensor. Based on the heartrate and the movement of the user 100 over a period of time, the mobile device 104 detects whether the user 100 is asleep or awake. The information about the mobile device 104 or the user 100 is used to provide information about or treat the diabetic condition.

The mobile device 104 provides information to the user 100 about the user's diabetic condition. For example, the mobile device 104 provides blood glucose levels, provides meal-related information, provides exercise-related information, generates graphs and other graphical user interfaces for display, or generates alerts that are provided to the user 100. For example, the mobile device 104 measures the blood glucose level of the user 100 and provides an alert when the blood glucose level of the user 100 has reached a threshold for an extreme diabetic state *(e.g.,* hypoglycemia or hyperglycemia). The alerts provided by the mobile device 104 are audible or non-audible alerts. The non-audible alerts are provided as a vibration, a flashing of the screen, or a flashing of an LED on the mobile device 104. In some embodiments, the alerts are also, or alternatively, provided by an external device based on a communication from the mobile device 104.

The mobile device 104 communicates with other devices directly via a wired communication or a short-range wireless communication (e.g., WI-FI^{®}, BLUETOOTH^{®}, BLE, NFC, or another short-range wireless communication). The mobile device 104 communicates indirectly with remote computing devices 122 or datastores 124 via a network 120 *(e.g.,* using a WI-FI^{®} network, a cellular network, a WI-MAX^{®} network, or another wired or wireless network). The network 120 is a wired or wireless network. The network 120 is used to communicate over the Internet to other devices.

The mobile device 104 communicates with the remote computing devices 122 to generate user interfaces for display on the mobile device 104, perform remote computation, or otherwise control a remote computing device. For example, the mobile device 104 provides a user interface via an application or web browser that is generated at a remote computing device 122. The mobile device 104 generates instructions for providing alerts via remote computing devices 122 based on information received from the user 100, the CGM 102, the BGM 106, or the insulin pump 116, 118. Example remote computing devices 122 to which the mobile device 104 sends communications for performing alerts include a remote computer *(e.g.,* a server, a laptop, or other computer), an external speaker, an external display device (e.g., television, monitor, or another device having an external display), a home automation system, a remote telecommunications device *(e.g.,* for sending text or voice communications to an emergency contact over a telecommunications network), a home appliance (e.g., refrigerator), or another remote computing device.

The mobile device 104 communicates with the datastores 124 to store information or retrieve information. The information includes information related to the user 100, the CGM 102, the BGM 106, or the insulin pump 116, 118. For example, the mobile device 104 receives treatment information associated with the user 100 as input or receive blood glucose information from the CGM 102 or the BGM 106 and send the information to the datastores 124 via the network 120. Stored information is retrieved from the datastores 124 for treatment of the diabetic condition of the user. For example, the mobile device 104 retrieves an amount of insulin delivered to the user 100 or corresponding times of delivery. The datastores 124 include one or more remote storage locations, which are collectively referred to as cloud storage. The datastores 124 include a lookup database of information on various food items, such as universal product codes (UPCs) or quick response (QR) codes, known or tagged images of the food items, or the nutritional information of the food items. The nutritional information of the food items includes, for example, the amount of calories, calories from fat, fat, saturated fat, trans fat, cholesterol, sodium, carbohydrates, dietary fiber, sugars, protein, vitamin A, vitamin C, calcium, iron, or the like.

If the user 100 is experiencing, or is about to experience, an extreme diabetic event, such as hypoglycemia, the user 100 may need to quickly ingest food items that are high in carbohydrates to increase blood glucose levels. If the user 100 is experiencing, or is about to experience, hypoglycemia or another extreme diabetic event (e.g., hyperglycemia), the user 100 may need to obtain diabetes-related supplies, such as a glucose testing lancet, a glucose testing strip, a blood glucose monitor, ketone testing supplies, meters, insulin, or seek medical assistance from a healthcare practitioner. If the user 100 is not carrying the appropriate food or supplies, the user 100 may need to quickly identify and locate a nearby source. If the user 100 is beginning to experience symptoms of an extreme diabetic event (e.g., confusion, disorientation, or lack of concentration), or if the user is an unfamiliar location *(e.g.,* an airport, a new city, or a town), the user 100 may have difficulty locating the appropriate food, supplies, or medical assistance.

The user devices described in connection with FIG. 1 assist the user 100 in locating sources of food items (e.g., carb-related food items). The user devices assist the user 100 during a diabetic-related event, which is detected by at least one of the user devices. For example, the mobile device 104 detects a hyperglycemic or hypoglycemic condition associated with the user 100 based on information from the CGM 102 or the BGM 106. The mobile device 104 detects that a supply of a diabetes medical product associated with the CGM 102, the BGM 106, or the insulin pump 116, 118 is below a predefined threshold. The mobile device 104 detects that the user 100 is seeking assistance. The mobile device 104 then obtains information regarding its location and information regarding the food items.

For example, the information regarding the food items includes a date or time of when the items were purchased by the user 100, available sources of the food items, locations of the sources of food items, or nutritional information associated with the food items (e.g., carbohydrates or glycemic index).

In some embodiments, the mobile device 104 displays the information in a predefined order or list. For example, the mobile device 104 displays a list of food items in order of their glycemic index or purchase date. In another example, the mobile device 104 establishes the list as a relative hierarchy for the time to access food items based on the type of establishment. The hierarchy can be established by providing a different weight or rank to different types of establishments, such as a food or beverage vending machine, a pharmacy with glucose tablets, a pharmacy with food for sale, a fast-food restaurant, a cafeteria, a grocery store, a sit-down full service restaurant, and other establishments. The weight or rank given to an establishment is based on the relative time periods calculated for the time to receipt of the food items.

The time to access the food items can include the travel time proximity to the user device and the time to access the food items. In an example embodiment, the time to access the food items includes a wait time after arriving at the source. For example, a wait time for receiving the food items, medical supplies, or medical services at an establishment is relatively higher for an establishment with more people located at the establishment than another. The wait time is based on current information or historical information regarding the number of people that have entered the establishment in a predefined period of time. The number of people waiting for service can be detected from the number of people that have entered the establishment and the number of people that have been served in the same time period. In another example, the number of people waiting for service is estimated based on the number of people that have been served by the establishment over a period of time *(e.g.,* the last hour, during a similar time of day during the week, or another predefined period of time). The mobile device 104 may access the wait times for different establishments from a datastore and use the wait times and the time of travel to calculate the time to receipt of food items, medical supplies, or medical services.

According to the claimed invention, if the user 100 is at home, the mobile device 104 aids the user 100 in locating carb-related food items in the user's refrigerator. The mobile device 104 or a processor in the refrigerator accesses the refrigerator's inventory of food items or the images from the refrigerator's camera(s) to identify carb-related food items therein. The carb-related food items are displayed on the mobile device 104 or on a display associated with the refrigerator.

The mobile device 104 also stores information to assist the user 100 in managing a diabetic condition. For example, the mobile device 104 stores a photo of the user's prescription(s) or information regarding the user's dosage, pharmacist or physician (e.g., names, addresses, phone numbers, or other information).

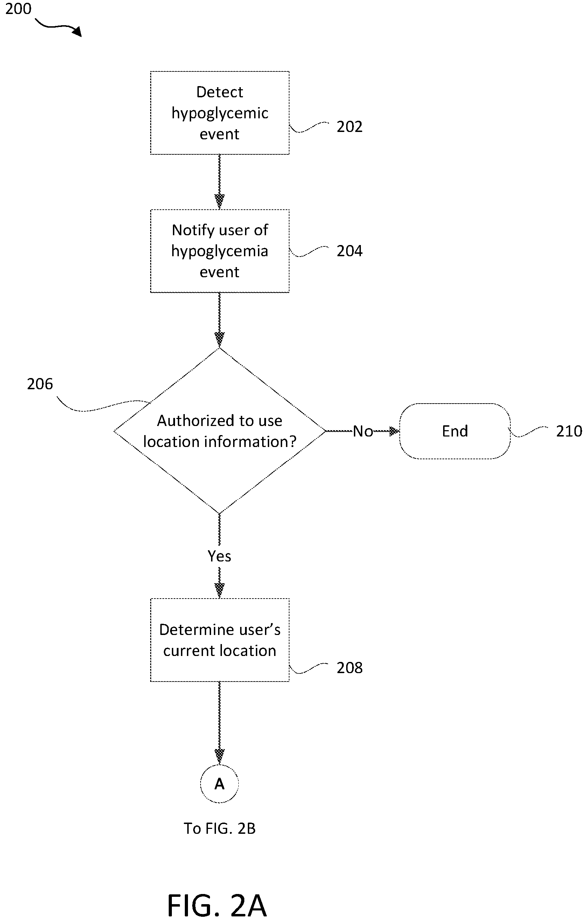

FIGs. 2A-2C illustrate a flow diagram of an example process 200 for assisting the user 100 in locating carb-related food items (*e.g.,* food with a predefined minimum amount of carbohydrates or food above a predefined glycemic index). The process 200 is implemented in one or more of the user devices described in connection with FIG. 1, such as the mobile device 104, the CGM 102, the BGM 106, the insulin pump 116, 118 or the remote computing device 122. The process 200, or portions thereof, is implemented in the user devices via a software application or firmware. The process 200 enables the user devices to, among other things, utilize location information to identify sources of carb-related food items that are near the user 100. For example, the mobile device 104 determines its location using global positioning system (GPS) signals, WI-FI^{®}, BLUETOOTH^{®} beacon signals, near-field communication (NFC) signals or other RF communication signals and identify sources of carb-related food items in close proximity. The location comprises a latitude and longitude, a street address, or a user-friendly name. The latitude and longitude are determined from the GPS signals and are compared to the latitude and longitude of a location on a map to determine the street address or user-friendly name.

As shown in FIG. 2A, at 202 of the process 200, a hypoglycemia event associated with the user 100 is detected. The event is detected based on information gathered from the CGM 102 or BGM 106, for example. For example, data from the CGM 102 or the BGM 106 indicates a hypoglycemic event. That is, the user 100 is experiencing a hypoglycemic event or is likely to experience a hypoglycemic event within a predefined period of time. The data is transmitted to the mobile device 104. At 204, the user 100 is notified of the hypoglycemic event via at least one user device. For example, the mobile device 104 displays a message, notification or image on a graphical user interface (GUI) alerting the user 100 of the hypoglycemic event.

At 206, the mobile device 104 determines whether the user 100 has authorized the use of the user's location information. The authorization information may be obtained from the user 100 as part of an initial set up of a mobile application on the mobile device 104, for example. The obtained authorization governs the subsequent use of the location information by the mobile device 104. Additionally, or alternatively, the mobile device 104 requests authorization each time the mobile device 104 attempts to use the location information of the user 100. If the user 100 has not provided authorization, the process 200 ends at 210. Otherwise, if the user 100 has provided authorization, the mobile device 104 determines the location of the user 100 at 208.

The mobile device 104 uses any suitable location service to determine location information related to the mobile device 104. The location service is used to determine latitude, longitude, altitude, a floor of a building, a street name, a city name, a country name, a postal code, a geographic feature or point of interest (*e.g*., names of mountains, rivers, businesses, or landmarks), or course information (*e.g*., speed or direction) associated with the mobile device 104. For example, the location services are accessed using Core Location, which includes parameters such as CLLocation, CLFloor, CLLocationManager, CLGeocoder, or CLPlacemark. The CLLocation parameter includes the latitude, longitude, altitude, or course information. The CLFloor parameter includes the floor of a building on which the mobile device 104 is located. The CLLocationManager parameter reports on location as a latitude/longitude pair. The CLGeocoder parameter facilitates the conversion between geographic coordinates and user-friendly names associated with the coordinates. The CLPlacemark parameter includes properties for specifying the street name, city name, country name, or postal code. The CLPlacemark includes properties describing relevant geographic features or points of interest at the location.

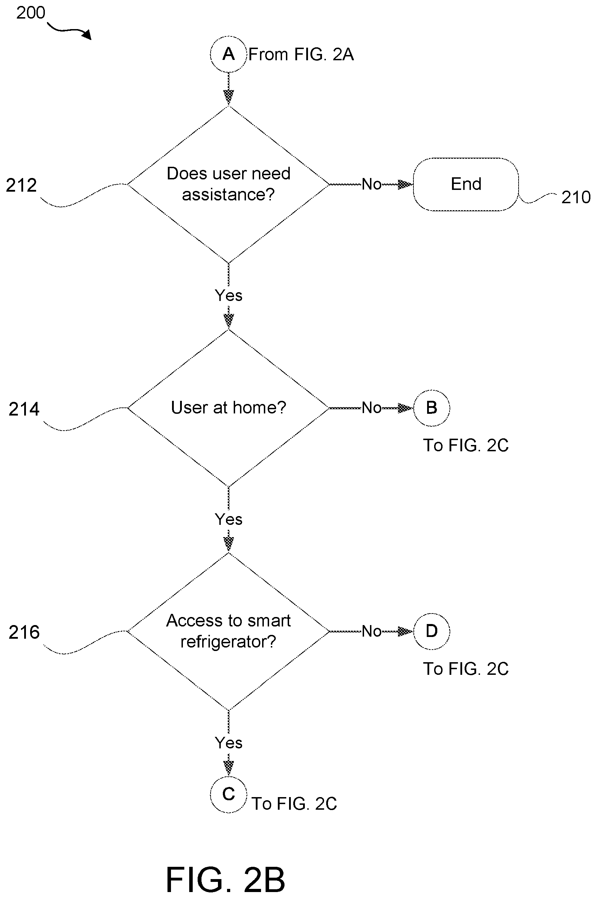

As shown in FIG. 2B, at 212, the mobile device 104 determines whether the user 100 needs assistance locating carb-related food items. For example, the mobile device 104 displays a message "Need help finding carbs?" on the GUI. If the user 100 provides an affirmative response (*e.g*., by selecting a "Yes" button on the GUI), the mobile device 104 proceeds to helping the user locate carb-related food items. For example, at 212, the mobile device 104 determines whether the user 100 is currently at a home location, such as the user's house or apartment. The mobile device 104 stores personal information or a personal profile of the user 100, such as birthday, age, home address, or phone number. The mobile device 104 determines that the user 100 is at a home location based on the coordinates of the home address and the current coordinates of the mobile device 104. The mobile device 104 also, or alternatively, determines that the user is at home based on a beacon ID received from the RF beacon device 126.

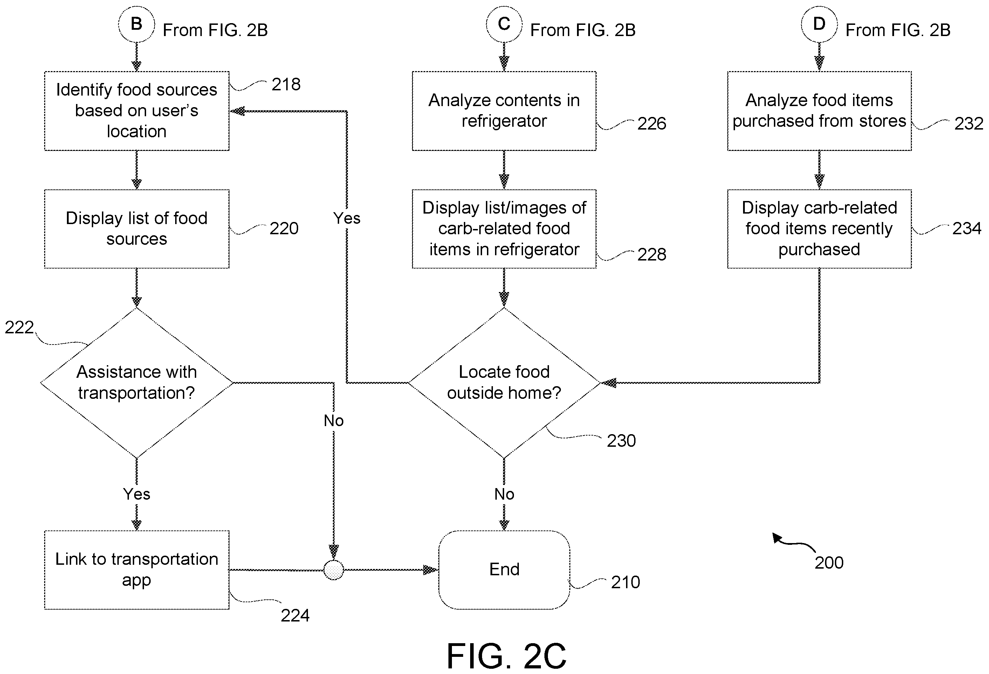

According to the claimed invention, if the mobile device 104 determines that the user 100 is located outside of the user's home, the mobile device 104 searches for the location(s) of carb-related food items within a predefined distance of the user's current location at 218, as shown in FIG. 2C. The mobile device 104 accesses an external location service to access establishment details about the establishments at the location(s) of carb-related food items within a predefined distance of the user's current location. The establishment details may include an identifier of the establishment, a latitude and longitude, a user-friendly name, a type of establishment, an address, hours of operation, indoor/outdoor identifier, an indicator of whether the location is permanently closed, accepted forms of payment, public accessibility, items sold (*e.g.,* food), a photograph, a phone number, a webpage, or other information about the establishment. Though each of these parameters is described as being received in or accessed from the establishment details, one or more of these parameters can be determined from other parameters. For example, the public accessibility, the items sold, or the accepted forms of payment can be determined or inferred from the identifier of the establishment, the user-friendly name, the type of establishment, or another parameter in the establishment details.

In an example embodiment, one or more parameters in the establishment details are used to identify places that sell food having carbs or a predefined number of carbs. For example, the identifier of the establishment, the user-friendly name, the type of establishment, the items sold, or another parameter in the establishment details may be used to identify places that sell food having carbs or a predefined number of carbs. The types of establishments could indicate a food or beverage vending machine, a pharmacy with glucose tablets, a pharmacy with food for sale, a fast food restaurant, a cafeteria, a grocery store, a sit-down full-service restaurant, or another type of establishment that indicates an establishment that sells carbs.

The mobile device 104 displays the relevant sources or respective locations within the predefined distance at 220. For example, the mobile device 104 identifies or displays a list of establishments (e.g., restaurants, grocery stores, convenience stores, or vending machines) where the user 100 may purchase or obtain carb-related food items. In some embodiments, the list of establishments is a filtered list based on predefined criteria. The filtered list includes a subset of the establishments that have been identified. In addition to displaying a list of establishments that can serve as a source of carbohydrates, the mobile device 104 optionally displays a recommendation for a serving size of a food item that provides a sufficient quantity of glucose to return the blood sugar level of the user 100 to an acceptable range. For example, in one embodiment the mobile device calculates a serving of sugar in grams that is required to raise the blood sugar level of the user 100 based on the blood sugar level measured by the CGM 102, BGM 106, or another blood glucose monitoring device along with stored profile data specific to the user 100 such as the weight, age, and gender of the user 100 to determine the required serving size of sugar. The mobile device 104 optionally displays the recommended serving of sugar to the user 100 as a direct measurement (e.g., a number of grams of sugar) and optionally identifies servings of common food items that contain the recommended amount of sugar. For example, a 10-gram serving of sugar could corresponding to one-half of a commonly available candy bar, and the mobile device 104 identifies portions of commonly available food items that contain the necessary serving of sugar using a database stored either in the memory of the mobile phone 104 or one of the external data stores 124 that are accessed via the network 120. The mobile device 104 optionally displays the recommended serving of commonly available food items to the user 100 to simplify the process of obtaining the appropriate amount of sugar to treat the hypoglycemic condition.

One or more of the listed establishments may be inaccessible to the public. In an example, establishments are stored in the datastore with identification parameters, such as the identifier of the establishment, the user-friendly name, or the type of establishment. Based on one or more of the identification parameters, the public accessibility is determined by the user 100 or the mobile device 104. For example, an establishment having a type of establishment that is listed as a park or library is determined to be accessible to the public, while a type of establishment such as a private residence or corporation is determined to be inaccessible to the public. In another example, an establishment is identified as an employee cafeteria that is inaccessible to non-employees from the identifier of the establishment, the user-friendly name, or the type of establishment. This may be intuitively discernible to the user 100 by examining the identifier of the establishment, the user-friendly name, or the type of establishment identified by the mobile device 104. The inaccessibility may also, or alternatively, be indicated by the public accessibility received in the establishment details. The listed establishments that are inaccessible to the public are indicated as such on the GUI to help guide the user100. The user 100 may still attempt to purchase or obtain food from the establishment (*e.g*., due to the urgent nature of the user's condition) or the user 100 may eliminate the establishment from consideration and move to the next establishment on the list. The establishments that are inaccessible to the public are filtered from the list of establishments, unless the establishment is associated with the user 100 (*e.g.,* the address is identified as a work address associated with the user in storage).

In some embodiments, the mobile device 104 filters the list of establishments based on one or more other criteria to display a subset of the establishments. For example, the mobile device 104 filters out establishments that are currently closed or otherwise inaccessible to the user 100. In some embodiments, the filter is based on the hours of operation in the establishment details and the current time, or the indication that the establishment is permanently closed. The mobile device 104 displays or list the establishments based on the user's payment preferences, such as a payment type, and the acceptable forms of payment in the establishment information. For example, if the user 100 prefers to pay with credit, a type of credit card, or otherwise does not have cash on hand, the mobile device 104 displays establishments that take credit, or the type of credit card, and filter out establishments that fail to accept credit, or the type of credit card, as a form of payment.

In some embodiments, the mobile device 104 filters results based on whether the establishment is an indoor or outdoor location. The mobile device 104 determines that the establishment is an indoor location based on the establishment having the same address as the address of the mobile device 104, the establishment type, or the establishment being identified based on short-range RF communications (*e.g*., BLE, NFC, Wi-Fi, or other short-range RF communications). The mobile device 104 also, or alternatively, accesses an electronic map of the facility in which the mobile device 104 is located and identifies the establishment as being located on the electronic map. As an option, the mobile device 104 omits outdoor establishments from the list when there is an indoor source of carbohydrates to prevent the user from having to travel outdoors when predefined weather conditions are met. The mobile device 104 accesses weather information from a weather service and omits the outdoor establishments due to poor weather conditions (*e.g*., temperature, precipitation, or other weather conditions) when an indoor location is available. The outdoor establishments are indicated by the mobile device 104 when they are closer or able to be reached more quickly than the indoor establishments.

In some embodiments, the mobile device 104 filters by the type of food that is available or omits establishments that sell food that is low in sugar or carbohydrates (*e.g*., diet beverages). The type of food that is available may be received in the establishment information, or the mobile device 104 queries a datastore 124 associated with the establishment identified in the establishment information to receive the items sold at the establishment. The nutritional value of the type of food is identified based on the food type *(e.g.,* diet soda) or the name of the food, and the establishment that is limited to selling types of food that are low in sugar or carbohydrates (*e.g*., diet beverages) is filtered out. The items sold at an establishment are identified and compared to a dataset (e.g., database) that has associated carbohydrates or glycemic index stored with one or more of the items sold. The mobile device 104 filters/sorts the list of establishments by the establishments that sell items above a predefined number of carbohydrates or glycemic index.

It is important for the user 100 to address the hypoglycemic event as quickly as possible. As such, the mobile device 104 prioritizes establishments that are in a predefined proximity over other criteria. The mobile device 104 may receive a travel time to the establishment from the external location service, or determine the travel time based on the distance from the current location of the mobile device 104 and the traffic conditions corresponding to the selected mode of transportation (*e.g.,* foot, car, public transportation, taxi, or ride share). For example, if the user 100 is indoors but the closest indoor food source is a three-minute walk, while the closest outdoor food source is a thirty-second walk, the mobile device 104 ranks the outdoor source above the indoor food source given that it takes the user 100 less time to reach the outdoor food source. The mobile device 104 receives the travel time to the establishment from the external location service in response to the current location of the mobile device 104, the location of the establishment, or a mode of transportation (*e.g*., foot, car, public transportation, taxi, or ride share).

The mobile device 104 determines the travel time to an indoor location based on the distance to the indoor location according to an electronic map of a building. In some embodiments, the electronic map of the building is received from a datastore 124. The travel time is determined based on an estimation of the user's walking speed and the distance on the electronic map. In some embodiments, the indoor location of the food source is identified as being on a floor of the building. The mobile device 104 identifies the floor on which the device is currently located (e.g., using the CLFloor parameter) to direct the user 100 to the closest establishment on the current floor or nearby floors. The travel time takes into account the distance the user 100 travels across each floor of the building to access the food source.

When displaying or listing nearby sources of carb-related food items, the mobile device 104 includes establishment information, such as the names of the establishments, respective distances, indoor/outdoor identifier, friendly-name address or location, or other information, such as a picture of the front of the establishment or a (e.g., miniature) map with a highlighted route. If the user 100 selects one of the establishments via the GUI on the mobile device 104, the mobile device 104 provides directions or open a native map/directions application. In some embodiments, the establishment information (e.g., the identifier of the establishment, the user-friendly name, or the type of establishment, the address) of the selected establishment is provided to the native map/directions application from another application executing on the mobile device 104, such as a diabetes treatment application (e.g., a diabetes data logging application, a bolus calculator application, a CGM application, or the like). Additionally, or alternatively, the mobile device 104 hands over the criteria to locate one or more identified establishments (e.g., the list of establishments or a selected establishment) to the native map/directions application, which then directs the user 100 to one or more of the establishments. The criteria include the type of establishment (e.g., restaurants, grocery stores, convenience stores, or vending machines), the current location of the mobile device 104, or a predefined distance for identifying establishments.

Referring to FIG. 2C, at 222, the mobile device 104 asks the user 100 if assistance with transportation is desired or needed. For example, the mobile device 104 indicates the distance to an establishment or the travel time by a mode of transportation (*e.g*., foot, car, public transportation, taxi, or ride share). The mobile device 104 arranges or ranks the establishments according to the travel time, such as in an ascending order. The user 100 may also be able to filter the results on the mobile device 104 according to the preferred mode of transportation. If the user 100 indicates (*e.g*., via the GUI) at 222 that assistance with transportation is desired, the mobile device 104 provides a link to a transportation application at 224. For example, if the user 100 wishes to arrange transportation via a ride sharing service (*e.g.,* a car share service, a scooter or bike rental service, or another ride sharing service), the mobile device 104 provides a link to such a service. When the transportation service is provided via another application, the location of the mobile device 104 or the establishment information is provided to the application for the transportation service. If the user 100 wishes to take public transportation, the mobile device 104 provides a link to a site that identifies the appropriate train or bus to board as well as scheduled departure times. After the user 100 arranges transportation, or if the user 100 indicates that assistance with transportation is unnecessary at 222, the process 200 ends at 210.

Referring again to 214 in FIG. 2B, if the mobile device 104 determines that the user 100 is at home at 214, the mobile device 104 identifies carb-related food items within the home. For example, the mobile device 104 identifies recently purchased carb-related food items, or carb-related food items in the refrigerator. The mobile device 104 determines if the user 100 owns or has access to a refrigerator, such as a smart or family hub refrigerator, at 216. The refrigerator includes one or more cameras for creating images of the internal area of the refrigerator. The images are created at predefined times, such as when the user 100 opens or closes the refrigerator door. The images are stored in the refrigerator's memory. The images may be viewed by the user 100 via a display on the door of the refrigerator, for example. The images enable the user 100 to visually see the specific items in the refrigerator and their relative locations therein. The refrigerator includes wireless connectivity, such as a cellular, Wi-Fi, or BLUETOOTH^{®} chipset, which enables the refrigerator to communicate its list of contents or images to the other user devices, such as the mobile device 104.

As shown in FIG. 2C, if the user 100 owns or has access to a smart refrigerator, the mobile device 104 analyzes the items in the refrigerator at 226. For example, the mobile device 104 queries the refrigerator for the list of its items or internal images. At 228, the mobile device 104 displays the list or internal images. The displayed list or images are configured to specifically identify carb-related food items, such as non-diet soda, fruit juice, raisins or the like. For example, the refrigerator includes an inventory system with a UPC scanner or a QR code scanner. The user 100 may scan each item when placing it into, or when removing it from, the refrigerator. In some embodiments, the refrigerator is configured to identify the specific product using the UPC or QR code and a lookup database in the datastores 124. The lookup database in the datastores 124 includes nutritional information about the items in the refrigerator, such as the amount of carbohydrates or calories in the food. The mobile device 104 has access to the datastores 124. As such, the mobile device 104 is configured to use the inventory list and the nutritional information provided by the refrigerator, or provided by the datastores 124, to list or rank the food items (*e.g.,* the carb-related food items) in the refrigerator in order of their respective glycemic index, which reflects their glucose-raising efficacy. This helps the user 100 to quickly identify specific glucose-raising items in the refrigerator. Additionally, as described above with reference to the processing of block 222, the mobile device 104 or a processor that is incorporated into the smart refrigerator can optionally identify and recommend a serving size of each available food item that provides a sufficient serving of sugar to treat the hypoglycemic condition in the user 100.

The mobile device 104 or the refrigerator's inventory system includes image recognition capabilities (*e.g*., machine vision or pattern matching functionality), which enables the identification of food items without the use of a scanner. For example, the camera(s) or processor(s) (*e.g*., an internal processor or via cloud computing) in the mobile device 104 or the refrigerator are configured to identify the specific items in the images generate at the refrigerator by matching visual markings on the items. In some embodiments, this is implemented via a two-dimensional array that matches captured images to known images of specific objects. The mobile device 104 or the refrigerator can have access to a neural network or other machine-learning image-recognition service capable of performing image-recognition. The image-recognition service is accessed via an application programming interface (API) capable of making function calls to a machine-learning image-recognition service that performs recognition of predefined items in an image. The image of an object is identified as being of the same type of object as the known image of an object when a threshold level of similar features is detected between the images. As such, the camera(s) or processor(s) are configured to identify the items in the images, including their respective locations within the refrigerator. As noted above, the mobile device 104 or the refrigerator has access to a lookup database that provides nutritional information about the identified items. By accessing the refrigerator's inventory system or the lookup database in the datastores 124, the mobile device 104 provides or display a list of carb-related food items in the refrigerator. The list is ranked according to the items' glucose index, which indicates the glucose-raising efficacy. The list includes a name, miniature picture, or location of each item. For example, a carb-related food item is circled or otherwise highlighted in an image displayed by the mobile device 104. Additionally, or alternatively, the list includes a textual description of the location, such as lowest shelf at front or left door - top shelf.

At 230, the mobile device 104 asks the user if the user 100 would like assistance locating sources of food outside of the home. If the user 100 indicates that assistance is desired, or if the mobile device 104 or the refrigerator determine there are no carb-related food items in the refrigerator (*e.g*., the items are below a predefined glycemic index), the process 200 proceeds to 218, 220, 222 or 224, as described above. Otherwise, if the user 100 indicates that assistance with locating food outside of the home is not desired, the process 200 ends at 210.

In addition to, or alternative to, identifying carb-related food items in the refrigerator, the mobile device 104 identifies recently purchased carb-related food items. For example, referring again to 216 in FIG. 2B, if the mobile device 104 determines that the user 100 does not own or have access to a smart refrigerator the mobile device 104 analyzes the user's recent food purchases at 232, as shown in FIG. 2C. The purchases are compiled from the internet-linked history of grocery or convenience store purchases covering a predefined period of time. The purchases are accessed from a grocery list of items indicated as purchased by the user 100. The grocery list is stored locally on the mobile device 104 or obtained from a remote datastore 124. The purchases are filtered by the mobile device 104 to show a subset that includes the carb-related food items in block 234. For example, the recently purchased food items are identified and compared to a dataset (*e.g*., database) that has associated carbohydrates or glycemic index stored for one or more of the items on the list. The mobile device 104 filters/sorts the list of purchased items by a predefined number of carbohydrates or glycemic index. The list is displayed on the GUI of the mobile device 104. Additionally, as described above with reference to the processing of blocks 220 and 228, in block 234 the mobile device 104 can optionally identify and recommend a serving size of each recently purchased food item that provides a sufficient serving of sugar to treat the hypoglycemic condition in the user 100. Further, as described above with reference to the processing of blocks 220 and 228, in block 234 the mobile device 104 can optionally identify and display a score that indicates a diabetic quality of the food that is purchased. The score may be a relative score, such as a percentage out of one hundred or "High", "Medium" and "Low".

The purchases are listed or ranked according to transaction dates. For example, older purchases have a higher likelihood of having already been consumed by the user 100 than more recent purchases. Accordingly, food items purchased more recently are ranked higher than food items with older purchase dates. The list may help the user 100 to remember the food items already purchased and the items that are likely to still be available.

After displaying food items based on the user's purchase history, the process 200 proceeds to 230, where the mobile device 104 asks the user 100 if assistance with locating sources of food outside of the home is desired. If the user 100 indicates yes *(e.g.,* via the GUI), or if no carb-related food items were purchased within the predefined period of time *(e.g.,* the purchased items are below a predefined number of carbohydrates or glycemic index), the process 200 then proceeds to 218, 220, 222 or 224 as described above. Otherwise, if the user 100 indicates no, the process 200 ends at 210.

While the use of the food purchase history has been described in the context of the user 100 not owning or having access to a smart refrigerator, it will be appreciated that the mobile device 104 is configured to utilize the user's food purchase history in combination with information from the smart refrigerator to help the user 100 identify sources of carbohydrates that the user 100 already has in the home.

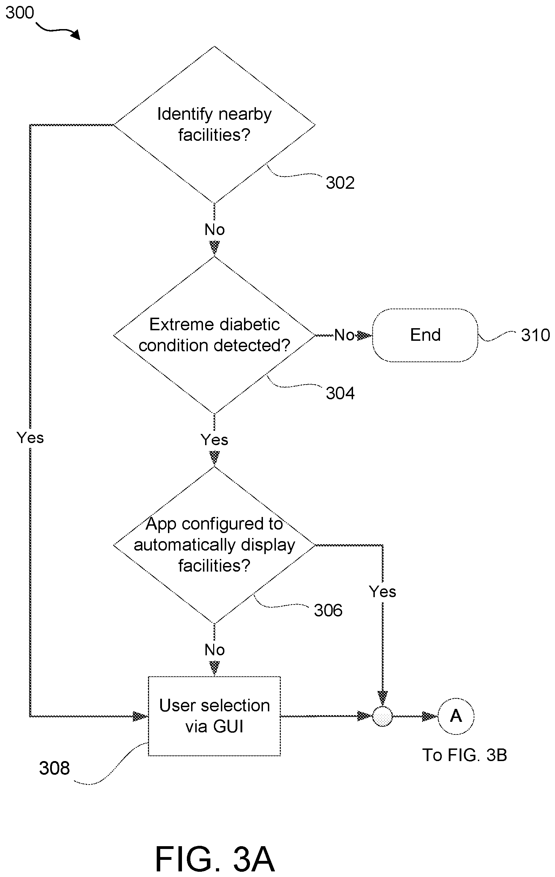

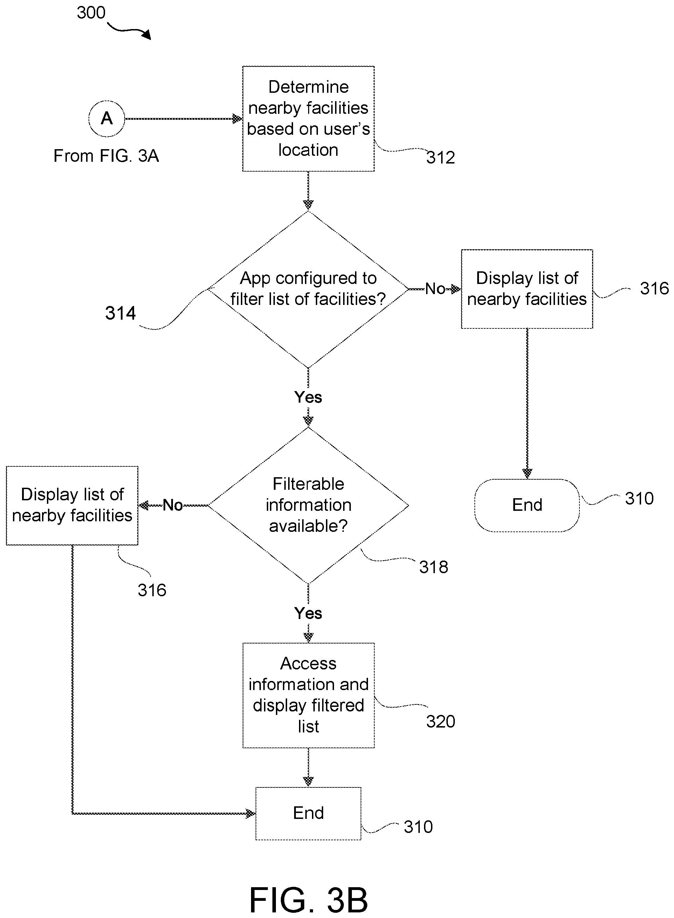

FIGs. 3A and 3B illustrate a flow diagram of an non claimed process 300 for assisting the user 100 in locating establishments that are sources of medical supplies (*e.g*., diabetes medical supplies facilities) or medical assistance (*e.g*., medical treatment facilities). For example, the user 100 may want to locate a source of medical supplies or medical assistance when a carb-related item is difficult to access. Like the process 200, the process 300 is implemented via a mobile application on the mobile device 104, for example. The processes 200, 300 are implemented via the same mobile application or via separate applications.

The mobile device 104 utilizes its location information to identify establishments that are sources of medical supplies or medical assistance that are within a predefined distance of the mobile device 104 of the user 100. As shown in FIG. 3A, at 302, a determination is made as to whether to identify a list of facilities that sell medical supplies (*e.g*., glucose testing lancet, a glucose testing strip, insulin, ketone testing supplies, meters, insulin, or CGM sensors) for diabetes medical devices (*e.g*., insulin pump or blood glucose meter) or facilities that can provide medical assistance to the user 100 *(e.g.,* an urgent care clinic, an emergency room, a hospital, a convenient care or retail clinic, a pharmacy, a mobile care clinic, or a primary care clinic.). The determination at 302 is made based on a triggering event, such as an indication from the user 100 on the mobile device 104 or an automatically detected event on the mobile device 104. For example, the mobile device 104 (*e.g.,* automatically) queries the user 100 regarding locating medical supplies based on a detected event from another user device, such as when a supply of a diabetes medical product associated with a diabetes medical device is below a predefined threshold (*e.g*., the insulin pump 116, 118 is below a predefined threshold or out of insulin or the BGM 106 is below a predefined threshold number or out of test strips). The mobile device 104 displays a notification on the GUI (*e.g.,* "You have no more insulin") or shows a button asking the user 100 if assistance with locating facilities or supplies is desired. The mobile device 104 (*e.g.,* automatically) shows the list of relevant facilities (*e.g.,* supply retail locations) as part of the notification on the GUI.

At 308, the user 100 may select an action via the GUI on the mobile device 104 for causing the mobile device 104 to identify establishments that are facilities within a predefined distance that are sources of medical supplies or medical assistance (*e.g*., tap on a "Find medical facilities" button or a "Locate medical supplies" button). In some embodiments, the sources of medical supplies are sources of supplies for a diabetes medical device. For example, the sources of medical supplies include insulin for an insulin pump, test strips for a blood glucose meter, or the like.

At 312, shown in FIG. 3B, the mobile device 104 determines the facilities within a predefined distance of the mobile device 104 based on the current location of the mobile device 104. As described herein, the mobile device 104 accesses an external location service to access establishment details about the establishments at the location(s) that are sources of medical supplies or medical assistance and within a predefined distance of the user's current location. The establishment details may include an identifier of the establishment, a user-friendly name, a type of establishment, an address, hours of operation, an indicator of whether the location is permanently closed, accepted forms of payment, items sold (*e.g.,* food, medical supplies, or medical services), an inventory (*e.g*., number or range) of items for sale, a photograph, a phone number, a webpage, or other information about the establishment. The establishment details are received from a datastore 124 associated with the establishment. For example, the establishment may have a datastore that is queried based on the identifier of the establishment, a user-friendly name, or an address. In response, the mobile device 104 receives establishment details that are maintained for the particular establishment, such as the hours of operation, accepted forms of payment, items sold (*e.g.,* food, medical supplies, or medical services), an inventory of items for sale, a phone number, a webpage, or other information about the establishment.

Referring again to FIG. 3A, if the triggering event is not detected for identifying the nearby facilities at 302, at 304 the mobile device 104 determines whether an extreme diabetic event (*e.g.,* a hyperglycemic or hypoglycemic event) has been detected. The extreme diabetic event is detected based on information from a blood glucose monitoring device, such as the CGM 102 or the BGM 106. The extreme diabetic event indicates whether the user 100 is currently experiencing a hyperglycemic or hypoglycemic event, or whether the user 100 is likely to experience such an event within a predefined period. If such an event is not detected, the process 300 ends at 310. Otherwise, if a hyperglycemic or hypoglycemic event is detected, at 306, the mobile device 104 determines whether it is configured to automatically display a list of nearby facilities after detecting a hyperglycemic or hypoglycemic event. If the mobile device 104 is not configured to automatically display a list of nearby facilities, at 308 the user 100 may make the appropriate selection via the GUI on the mobile device 104 (*e.g.,* tap on the "Find medical facilities" button or the "Locate medical supplies" button). If the user 100 makes the selection at 308, or if the mobile device 104 determines that it is configured to automatically display a list of nearby facilities at 306, the mobile device 104 determines a list of facilities within a predefined distance of the mobile device 104 based on the current location of the medical device 104 at 312, as shown in FIG. 3B.

Referring again to FIG. 3B, at 314, the mobile device 104 determines whether it is configured to filter the list of facilities in accordance with one or more user preferences. For example, the mobile device 104 stores one or more preferences selected by the user 100, such as preferences from the establishment information described herein. For example, the mobile device stores preferences regarding a name of a preferred facility, a name of a preferred facility type, a preferred travel distance, a desired item type (*e.g.,* type of medical supplies or type of medical services), a desired inventory for an item type, or predefined brand of medical diabetes product, desired operating hours, or the like. If the mobile device 104 is not configured to filter information regarding the facilities, the mobile device 104 displays the list of facilities within the predefined distance at 316. The process 300 then ends at 310.

If the mobile device 104 is configured to filter information regarding nearby facilities to generate a subset of facilities, the mobile device 104 determines whether or what filterable information is available at 318, such as the names of stores, respective inventories, or operating hours. If filterable information is available, the mobile device 104 displays a list of facilities in accordance with the user's desired filter at 320. For example, if the filterable information includes store inventories, the mobile device 104 determines which of the identified facilities stock a predefined inventory of diabetes medical supplies. The mobile device 104 applies the filter to list an amount of the diabetes medical supplies in stock at each of the facilities based on information from a respective dataset of diabetes medical supplies for each of the facilities. The mobile device 104 displays those facilities that stock a particular diabetes supply or product that the user 100 is seeking. Otherwise, if filterable information is unavailable, the mobile device 104 displays a list of nearby facilities without any filter (*e.g.,* display a list of facilities within a predefined distance). The process 300 then ends at 310.

The mobile device 104 stores personal information about the user 100 that is useful at the medical supplies or medical services facilities. For example, the mobile device 104 stores photographs of at least one of the user's prescriptions, and photographs or other data including the dosage, proper name, recent prescription refills, or phone numbers of either or both of a doctor or pharmacy. The personal information may help the user 100 more quickly access a medical supply or service.

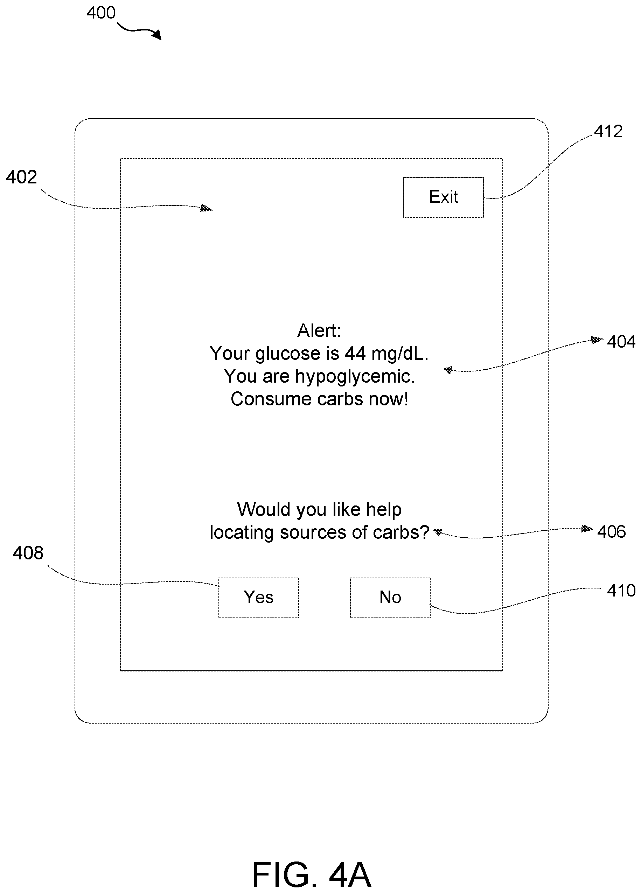

FIGs. 4A-4E depict example GUIs on a display 400 of the user device, such as the mobile device 104, the CGM 102, a CGM controller, the BGM 106, the insulin pump 116, 118 or the remote computing devices 122 *(e.g.,* a refrigerator). The GUIs are generated by software or firmware on the user device, such as a CGM application on the mobile device 104 or firmware in a refrigerator. The GUIs are generated locally, or on a remote device and then displayed via an application, such as a web browser, on the user device.

As shown in FIG. 4A, a GUI 402 is displayed on the display 400 *(e.g.,* the mobile device 104). The GUI 402 includes an alert 404. The alert 404 indicates a blood glucose level of the user 100, an indication of a current or predicted extreme diabetic state (e.g., hypoglycemia) associated with the user 100, or an indication to the user 100 to consume carbohydrates or insulin to help self-treat the extreme diabetic state. Though the alert indicates that the user 100 is hypoglycemic, in some embodiments, the alert is generated in response to the blood glucose level of the user 100 reaching a threshold prior to an extreme diabetic state.

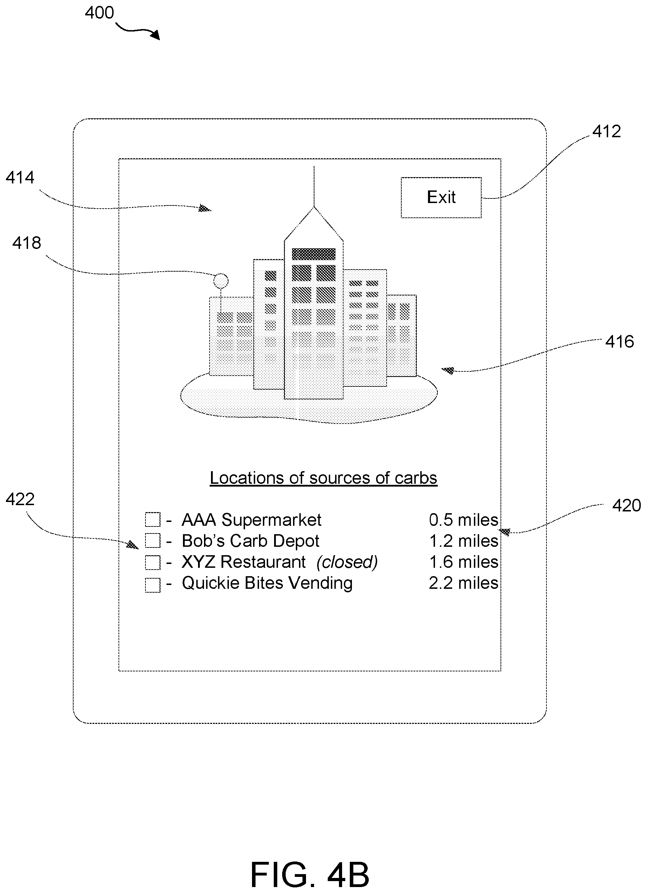

The GUI 402 includes a request 406, which asks whether the user 100 would like help locating sources of carbohydrates. Additionally, or alternatively, the request 406 asks whether the user 100 would like help locating sources of diabetes medical supplies or medical assistance. The user 100 may respond by selecting a Yes button 408 or a No button 410. Selecting the Yes button 408 takes the user 100 to another GUI, such as those described in connection with FIGs. 4B-4E below. The no button 410 takes the user 100 back to another GUI in the application that generated the alert.

The GUI 402 includes an exit button 412 that allows the user 100 to exit the screen on the GUI 402 (*e.g*., without answering the request 406) or exit the application. If the user 100 selects the exit button 412 or does not answer the request 406 within a predefined period of time, a diabetes-related alert is communicated from the user device (*e.g.,* from the mobile device 104) to solicit assistance for the user 100. It is assumed that the user 100 is incapable of answering the request 406.

As shown in FIG. 4B, a GUI 414 is displayed on the user device (*e.g.,* the mobile device 104). For example, the GUI 414 is displayed on the user device when the user device is determined to be outside of a home location of the user 100 to assist the user 100 in identifying sources of carbohydrates outside of the user's home. The GUI 414 includes a map 416, which includes a graphical representation of a geographical area in which the user device (*e.g.,* the mobile device 104) is located. The map 416 includes one or more location pins 418 for visually identifying the locations of the user 100 or sources of carb-related food items on the map 416. The location pins 418 indicate a location within a building, such as a floor or a room.

The map 416 includes a list 420, which includes the names of establishments that offer carb-related food items or the distance thereto. The list 420 includes the travel time to each establishment by transportation type, such as by foot, car, public transportation, or ride share. The list 420 indicates whether the establishments are currently open or closed. Each of the establishments in the list 420 corresponds to a respective location pin 418 on the map 416. The user 100 may select one of the establishments via a corresponding selection box 422. Upon selection, the GUI 414 presents detailed (*e.g*., text-based or GUI-based) directions to the establishment or automatically dial a phone number associated with the establishment. The user 100 may select the exit button 412 to exit the screen on the GUI 414.

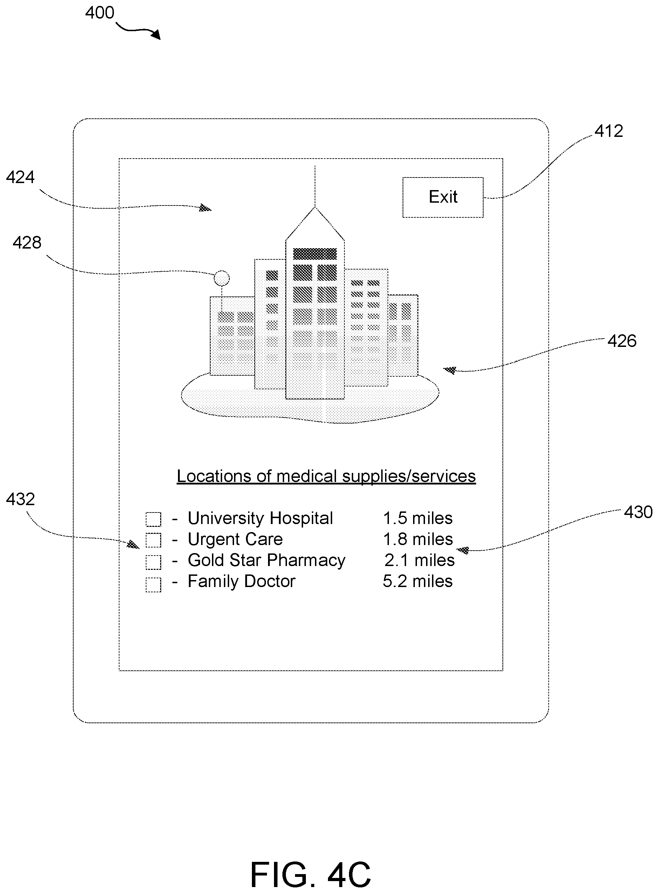

As shown in FIG. 4C, a GUI 424 is displayed on the user device (*e.g.,* the mobile device 104). For an example outside the claimed invention, the GUI 424 is displayed on the user device when the user device is identifying a medical supply or medical treatment facility. The GUI 424 includes a map 426, which includes a graphical representation of a geographical area in which the user device (*e.g.,* the mobile device 104) is located. The map 426 includes one or more location pins 428 for visually identifying the locations of diabetes medical supplies or medical services on the map 426. The map 426 includes a list 430, which includes the names of the facilities that offer diabetes medical supplies or medical services. The list 430 includes the distance or travel time according to a transportation type (*e.g.,* by foot, car, public transportation, or ride share) to each facility.

The list 430 includes inventory information, such as the quantity available of a certain medical supply at a facility. The list 430 also indicates indoor/outdoor locations. Each of the facilities in the list 430 corresponds to a respective location pin 428 on the map 426. The user 100 may select one of the facilities via the corresponding selection box 432. Upon selection, the GUI 424 presents detailed (e.g., text-based) directions to the facility or automatically dials a phone number associated with the facility. The user 100 may select the exit button 412 to exit the screen on the GUI 424.

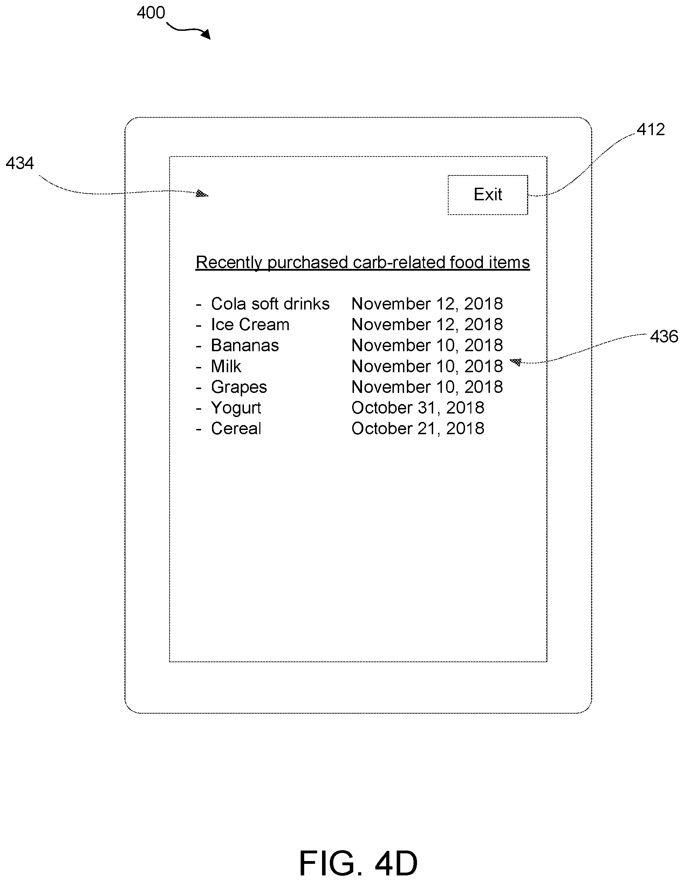

As shown in FIG. 4D, a GUI 434 is displayed on the user device (*e.g.,* the mobile device 104). For example, the GUI 414 is displayed on the user device when the user device is determined to be inside of a home location of the user 100 to assist the user 100 in identifying sources of carbohydrates inside the user's home. The GUI 434 includes a list 436. The list 436 includes recently purchased carb-related food items. The carb-related food items are ordered according to their respective transaction dates. For example, items purchased 2 days ago are listed higher than items purchased 4 days ago. The user 100 may select the exit button 412 to exit the screen on the GUI 434.

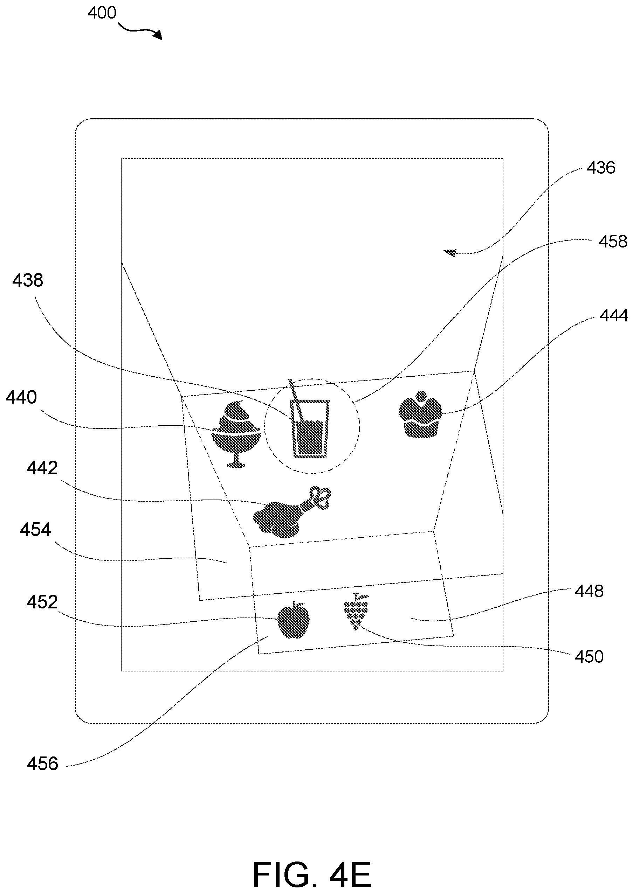

As shown in FIG. 4E, a GUI 436 is displayed on the user device (*e.g.,* the mobile device 104). For example, the GUI 414 is displayed on the user device when the user device is determined to be inside of a home location of the user 100 to assist the user 100 in identifying sources of carbohydrates inside the user's home. The GUI 436 depicts an internal area of a refrigerator in the home or apartment of the user 100. The internal area is captured by one or more cameras in the refrigerator. The GUI 436 includes shelves 454, 456, which include one or more food items. For example, the items on the shelf 454 include ice cream 440, chicken 442, a soft drink 438, or a cupcake 444. The items on the shelf 456 include, for example, bananas 448, grapes 450, or an apple 452. In some embodiments, the GUI 436 generates a photograph or a live video image of the item.

The display 400 is displayed on the mobile device 104, which communicates with the refrigerator directly via a wired communication or a short-range wireless communication (*e.g.*, WI-FI^{®}, BLUETOOTH^{®}, BLE, NFC, or other short-range wireless communication). In some embodiments, the mobile device 104 communicates indirectly with refrigerator via the network 120. As such, the GUI 436 is captured by the refrigerator's camera(s) and transmitted to the mobile device 104 for analysis or display.

In some embodiments, the display 400 is incorporated into the exterior side of a refrigerator's door, thereby enabling the user 100 to view the items in the refrigerator without having to open the door. In some embodiments, the refrigerator's inventory system includes a processor for executing image recognition software and includes access to a lookup database in the datastores 124. The lookup database includes information regarding UPC or QR codes or nutritional information related to food items. The lookup database includes known images of the food items. The processor compares one or more aspects of a captured image of a specific food item in the refrigerator to the known images in the datastores 124. If the processor identifies a match, the processor identifies the specific type or brand of the food item along with any nutritional information that is available.

The image recognition process in the refrigerator or the mobile device 104 includes the use of optical character recognition or an evaluation of colors or shapes associated with the food item. By using image recognition functionality, the refrigerator is able to create an inventory of the available food items, and their corresponding nutritional content, without input from the user 100. In some embodiments, the image recognition functionality is implemented on the mobile device 104, thereby enabling the mobile device 104 to identify or inventory the food items based on the image(s) it receives from the refrigerator. The mobile device 104 similarly identifies objects via a local camera when held in front of the refrigerator or when an image is taken with the camera. The image on the mobile device 104 is stored with a date, such that the most recent image is provided to the user, or the image is analyzed in real-time to provide the user 100 with indications of carb-related food sources.

The identification of food items via image recognition enables the refrigerator or the mobile device 104 to assist the user 100 in identifying food with certain nutritional content, such as foods that are high in carbohydrates or foods that are low in calories. For example, if the mobile device 104 detects a hypoglycemic event associated with the user 100, the mobile device 104 accesses the image(s) to determine if the refrigerator's contents include any carb-related food items. If the image recognition processing on the mobile device 104 results in the identification of carb-related food items, the mobile device 104 displays the relevant image (*e.g.,* the GUI 436) and visually call out the relevant carb-related food item.

For example, the mobile device 104 determines that the refrigerator contains the soft drink 438, which is deemed a carb-related food item having carbohydrates or a glycemic index that is above a predefined threshold. The mobile device 104 displays the GUI 436 with a call out feature 458 that highlights the soft drink 438. While FIG. 4E depicts the call out feature 458 in connection with the soft drink 438, In some embodiments, the call out feature 458 is used to highlight multiple carb-related food items, such as the ice cream 440 or the cupcake 444. As shown in FIG. 4E, the call out feature 458 includes a hashed circle around the soft drink 438, though any suitable visual effect for calling attention to the soft drink 438 may be used. For example, the call out feature 458 includes a solid line, a blinking line or lines with different colors, such as red, yellow, or green. In some embodiments, the call out feature 458 includes the ability to zoom in on the soft drink 438. The call out feature 458 helps the user 100 to quickly identify carb-related food items that are available and where they are located within the refrigerator.

As noted above, the display 400 is incorporated into the exterior side of the refrigerator door for viewing by the user 100. As such, if the mobile device 104 determines that the user 100 needs carb-related food items, the mobile device 104 signals the refrigerator (*e.g*., via the network 120) to determine if such items are available or to locate them. For example, the mobile device 104 requests the refrigerator to inventory its contents to identify carb-related food items, such as the soft drink 438. After identifying the soft drink 438, the refrigerator uses the call out feature 458 to call the user's attention to the soft drink 438 via the GUI 436 on the display 400.

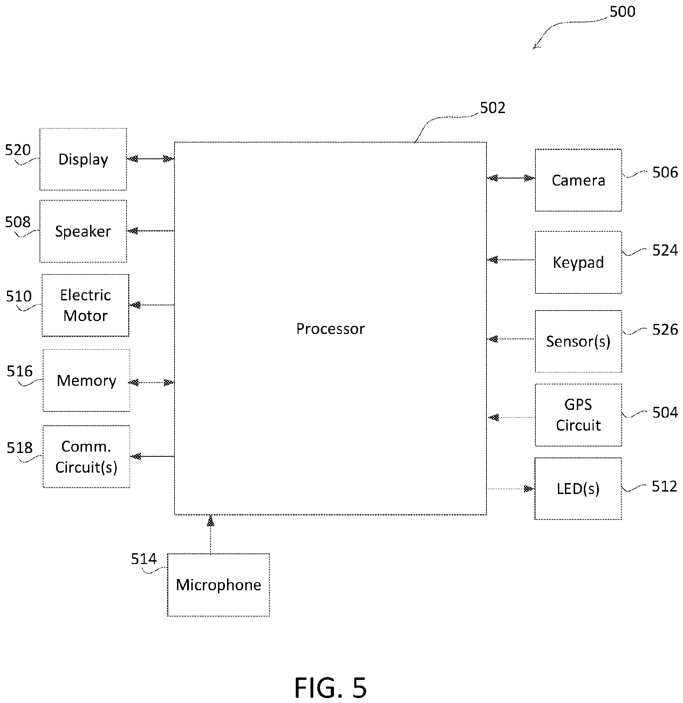

FIG. 5 is a block diagram of an example computing device 500. The computing device is a mobile computing device, such as a tablet, a cellular phone, a wearable device, a CGM controller device, or another computing device, for example. As shown in FIG. 5, the computing device 500 includes a processor 502 for controlling the functionality of the computing device 500. The processor 502 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 502 performs signal coding, data processing, power control, image processing, input/output processing, or any other functionality that enables the computing device 500 to perform as described herein.

The processor 502 stores information in or retrieve information from the memory 516. The memory 516 includes a non-removable memory or a removable memory. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 502 accesses the memory 516 for executable instructions or other information that is used by the computing device 500.

The computing device 500 includes a camera 506 that is in communication with the processor 502. The camera 506 is a digital camera or other optical device capable of generating images or videos (*e.g*., image sequences) for being captured at the computing device 500. The camera 506 includes a lighting device capable of flashing to in response to signals from the processor 502. The lighting device flashes to provide alerts via the camera 506.

The computing device 500 includes one or more communication circuits 518. The processor 502 is in electrical communication with the communication circuit 518 for sending or receiving information. The communication circuit 518 is capable of performing wired or wireless communications. For example, the communication circuit 518 includes one or more radio frequency (RF) transceivers for transmitting and receiving RF signals (e.g., BLUETOOTH^{®}, near field communication (NFC), WIFI^{®}, WI-MAX^{®}, cellular, or other RF signals) via an antenna, or other communications module capable of performing wireless communications. In some embodiments, one or more communication circuits 518 are capable of performing infrared (IR) communications.

The processor 502 is in electrical communication with a keypad 524 for providing input to the processor 502. The keypad 524 includes one or more keys for receiving input from a user. The keypad 524 includes hard or soft keys for which the function of the keys changes as a user performs selections.

Other input into the processor 502 is provided by one or more sensors 526. The sensors 526 include a motion sensor, a proximity sensor, a heartrate monitoring sensor, an accelerometer, a gyroscope, or another sensor on the computing device. The motion sensor transmits infrared signals or uses image processing to sense movement. The proximity sensor transmits infrared signals to detect when an object is within a predefined proximity. The heartrate monitoring sensor implements photoplethysmography to detect the amount of blood flow in the user. The heartrate monitoring sensor includes one or more LED or photodiodes to detect the amount of blood flow in the user. The heartrate monitoring sensor implements infrared technology to detect the amount of blood flow in the user. The heartrate monitoring sensor takes an electrocardiogram (ECG) and detects information about the user's heartrate from the ECG. The accelerometer measures the non-gravitational acceleration of the computing device 500 in a given direction. The accelerometer responds to vibrations associated with movement in a given direction. The measurements from the accelerometer are used by the processor 502 to determine the magnitude or direction of the relative movement of the computing device 500, or the user's relative position (e.g., standing, sitting, or lying down). The gyroscope is used to determine the orientation of the computing device 500.

The processor 502 is in electrical communication with or generate images on a display 520 for providing information to a user. The communication between the display 520 and the processor 502 is a two-way communication, as the display 520 includes a touch screen module capable of receiving information from a user and providing such information to the processor 502. For example, the display 520 provides soft buttons for selection by a user that are recognized by the touch screen module and provided to the processor 502 as input.

The processor 502 is in electrical communication with or control a speaker 508. The speaker 508 provides an audible sound *(e.g.,* tone, beep, or buzz) in response to a triggering event detected by the processor 502.

The computing device 500 includes an electric motor 510 that is in electrical communication with or controlled by the processor 502. The electric motor 510 rotates and causes the computing device 500 to vibrate (*e.g.,* to indicate an alert) in response to a triggering event detected by the processor 502. The electric motor 510 provides an alert to supplement the audible alarm or replace the audible alarm provided by the speaker 508.

The processor 502 is in electrical communication with or receive information from a microphone 514. For example, the processor 502 receives audio signals via the microphone 514.

The computing device 500 includes a global positioning system (GPS) circuit 504. The GPS circuit 504 is capable of receiving GPS information. The processor 502 is capable of determining the GPS coordinates (*e.g*., latitude and longitude) of the computing device 500 based on the GPS information received via the GPS circuit.

The computing device 500 includes a visual indicator, such as one or more light-emitting diodes (LEDs) 512. In some embodiments, one or more LEDs 512 are illuminated or flashed to provide an alert or communicate other information to the user (*e.g.,* low battery or turning on of the device)

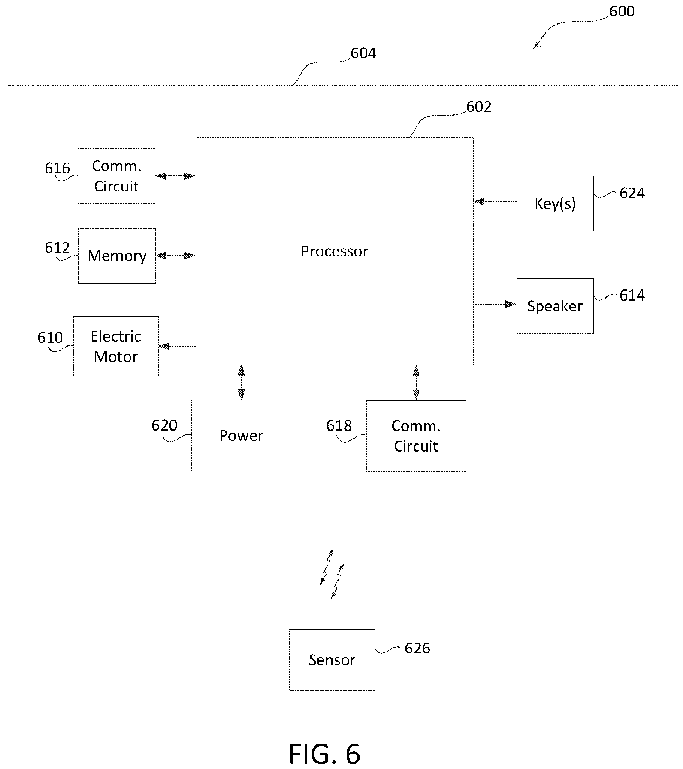

FIG. 6 is a block diagram of an example blood glucose monitoring device 600. In some embodiments, the blood glucose monitoring device 600 is a CGM or FGM, for example. The blood glucose monitoring device 600 includes a subcutaneous sensor 626 that is used to sense and monitor the amount of glucose in interstitial fluid of the user. Data is transmitted from the sensor 626 to a transmitting device 604. When the blood glucose monitoring device 600 is a CGM, the transmitting device 604 is located directly over the sensor 626 and wirelessly powers the data transfer from the sensor 626 via power supply 620. When the blood glucose monitoring device 600 is an FGM, the transmitting device 604 is a mobile device or other reader device that instantaneously receives the blood glucose information from the sensor 626 when the device is within the RF range of the sensor 626.

The transmitting device 604 receives data communications from the sensor 626 via a communication circuit 618. The communication circuit 618 is in electrical communication with a processor 602. The processor 602 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 602 performs signal coding, data processing, power control, input/output processing, or any other functionality that enables the transmitting device 604 to perform as described herein.

The transmitting device 604 includes another communication circuit 616 for communicating with other devices. The processor 602 is in electrical communication with the communication circuit 616 for sending or receiving information. The communication circuits 616, 618 are capable of performing wired or wireless communications. For example, the communication circuits 616, 618 include one or more radio frequency (RF) transceivers for transmitting and receiving RF signals (e.g., BLUETOOTH^{®}, near field communication (NFC), WIFI^{®}, WI-MAX^{®}, cellular, or other RF signals) via an antenna, or other communications module capable of performing wireless communications. The communication circuits 616, 618 communicate using the same RF protocol or a different RF protocol.

The processor 602 stores information in or retrieves information from the memory 612. The memory 612 includes a non-removable memory or a removable memory. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 602 accesses the memory 612 for executable instructions or other information that is used by the transmitting device 604. The processor 602 is in electrical communication with a one or more input keys 624 for providing input to the processor 602.

The processor 602 is in electrical communication with or control a speaker 614. The speaker 614 provides an audible sound (*e.g.,* tone, beep, or buzz) in response to a triggering event detected by the processor 602.

The blood glucose monitoring device 600 includes an electric motor 610 that is in electrical communication with or controlled by the processor 602. The electric motor 610 rotates and causes the blood glucose monitoring device 600 to vibrate (*e.g.,* to indicate an alert) in response to a triggering event detected by the processor 602. The electric motor 610 provides an alert to supplement the audible alarm or replace the audible alarm provided by the speaker 614.

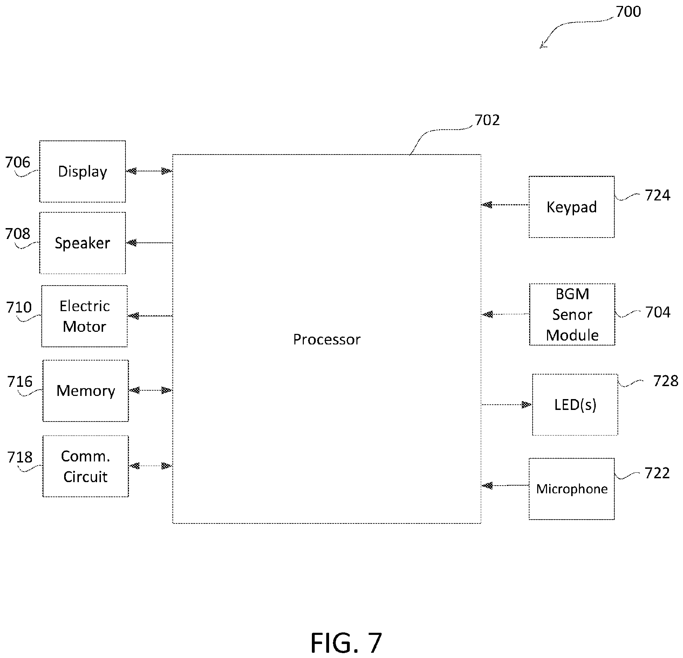

FIG. 7 is a block diagram of an example blood glucose measuring (BGM) device 700. As shown in FIG. 7, the BGM device 700 includes a processor 702 for controlling the functionality of the BGM device 700. The processor 702 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 702 performs signal coding, data processing, power control, image processing, input/output processing, or any other functionality that enables the BGM device 700 to perform as described herein.

The processor 702 stores information in or retrieve information from the memory 716. The memory 716 includes a non-removable memory or a removable memory. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 702 accesses the memory 716 for executable instructions or other information that is used by the BGM device 700.

The BGM device 700 includes one or more communication circuits 718. The processor 702 is in electrical communication with the communication circuit 718 for sending or receiving information. The communication circuit 718 is capable of performing wired or wireless communications. For example, the communication circuit 718 includes one or more radio frequency (RF) transceivers for transmitting and receiving RF signals (e.g., BLUETOOTH^{®}, near field communication (NFC), WIFI^{®}, WI-MAX^{®}, cellular, or other RF signals) via an antenna, or other communications module capable of performing wireless communications. In some embodiments, one or more communication circuits 718 are capable of performing infrared (IR) communications.

The processor 702 is in electrical communication with a keypad 724 for providing input to the processor 702. The keypad 724 includes one or more keys for receiving input from a user. The keypad 724 includes hard or soft keys for which the function of the keys changes as a user performs selections.

Other input into the processor 702 is provided by the BGM sensor module 704. The BGM sensor module 704 includes a blood glucose measuring engine that may analyze blood samples provided by a patient on a blood glucose measurement strip and measures the amount of blood glucose in the samples.

The processor 702 is in electrical communication with or generate images on a display 706 for providing information to a user. The communication between the display 706 and the processor 702 is a two-way communication, as the display 706 includes a touch screen module capable of receiving information from a user and providing such information to the processor 702. For example, the display 706 provides soft buttons for selection by a user that are recognized by the touch screen module and provided to the processor 702 as input.

The processor 702 is in electrical communication with or control a speaker 708. The speaker 708 provides an audible sound (*e.g.,* tone, beep, or buzz) in response to a triggering event detected by the processor 702.

The BGM device 700 include an electric motor 710 that is in electrical communication with or controlled by the processor 702. The electric motor 710 rotates and causes the BGM device 700 to vibrate (*e.g.,* to indicate an alert) in response to a triggering event detected by the processor 702. The electric motor 710 provides an alert to supplement the audible alarm or replace the audible alarm provided by the speaker 708.

The processor 702 is in electrical communication with or receive information from a microphone 722. For example, the processor 702 receives audio signals via the microphone 722.

The BGM device 700 includes a visual indicator, such as one or more one or more light-emitting diodes (LEDs) 728. In some embodiments, one or more LEDs 728 are illuminated or flashed to provide an alert or communicate other information to the user *(e.g.,* low battery or turning on of the device).

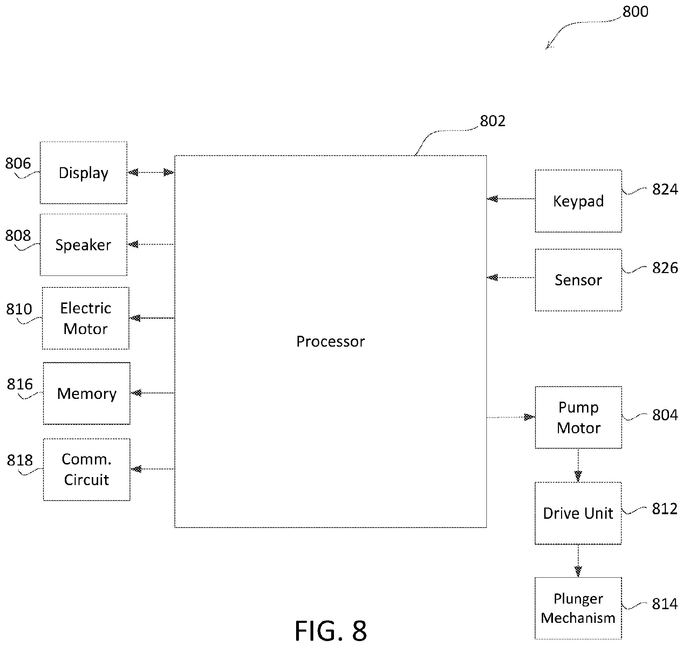

FIG. 8 is a block diagram illustrating an example of an insulin pump 800. As shown in FIG. 8, the insulin pump 800 includes a processor 802. The processor 802 includes one or more circuits, such as general-purpose processors, special purpose processors, conventional processors, digital signal processors (DSPs), microprocessors, integrated circuits, a programmable logic device (PLD), application specific integrated circuits (ASICs), or the like. The processor 802 performs signal coding, data processing, power control, image processing, input/output processing, or any other functionality that enables the insulin pump 800 to perform as described herein.

The processor 802 is in electrical communication with or control a pump motor 804 in the insulin pump 800. The pump motor 804 drives a drive unit 812 that pushes a plunger mechanism 814. The plunger mechanism 814 ejects insulin from an insulin cartridge (not shown). The insulin cartridge includes a supply of insulin for delivery to a user.

The processor 802 is in electrical communication with or generate images on a display 806 for providing information to a user. The communication between the display 806 and the processor 802 is a two-way communication, as the display 806 includes a touch screen module capable of receiving information from a user and providing such information to the processor 802. For example, the display 806 provides soft buttons for selection by a user that are recognized by the touch screen module and provided to the processor 802 as input.

The processor 802 is in electrical communication with or control a speaker 808. The speaker 808 provides an audible sound (*e.g.,* tone, beep, or buzz) in response to a triggering event detected by the processor 802.

The insulin pump 800 includes an electric motor 810 that is in electrical communication with or controlled by the processor 802. The electric motor 810 rotates and causes the insulin pump to vibrate (*e.g.,* to indicate an alert) in response to a triggering event detected by the processor 802. The electric motor 810 provides an alert to supplement the audible alarm or replace the audible alarm provided by the speaker 808.

The processor 802 is in electrical communication with a memory 816. The processor stores information in or retrieve information from the memory 816. The memory 816 includes a non-removable memory or a removable memory for storing computer-readable media. The non-removable memory includes random-access memory (RAM), read-only memory (ROM), a hard disk, or any other type of non-removable memory storage. The removable memory includes a subscriber identity module (SIM) card, a memory stick, a memory card (*e.g.,* a digital camera memory card), or any other type of removable memory. The processor 802 accesses the memory 816 for executable instructions or other information that is used by the insulin pump 800.

The insulin pump 800 includes a communication circuit 818. The processor 802 is in electrical communication with the communication circuit 818 for sending or receiving information. The communication circuit 818 is capable of performing wired or wireless communications. For example, the wireless communications circuit 818 includes a radio frequency (RF) transceiver for transmitting and receiving RF signals (e.g., BLUETOOTH^{®}, near field communication (NFC), WIFI^{®}, WI-MAX^{®}, cellular, or other RF signals) via an antenna, or other communications module capable of performing wireless communications. In some embodiments, the communication circuit 818 is capable of performing infrared (IR) communications.

The processor 802 is in electrical communication with a keypad 824 for providing input to the processor 802. The keypad 824 includes one or more keys for receiving input from a user. The keypad 824 includes hard or soft keys for which the function of the keys changes as a user performs selections.

Other input into the processor 802 is provided by sensors 826. The sensor 826 includes a pressure sensor that is sensitive to the pressure within a reservoir of insulin; a cartridge sensor that is sensitive to the presence of an insulin cartridge or a motion sensor that detects the motion of a gear (not shown) in the drive unit 812.

Although features, elements, and functions are described above in particular combinations, a feature, element, or function is used alone or in any combination with the other features, elements, or functions.

The methods described herein are implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random-access memory (RAM), removable disks, and optical media such as CD-ROM disks, and digital versatile disks (DVDs).

## Claims

1. A method comprising:
detecting, via information gathered from a blood glucose monitoring device (600), a hypoglycemic event associated with a user (100);
detecting a location of a mobile device (104) associated with the user (100) during the hypoglycemic event;
determining, by the mobile device (104), whether the user (100) is currently at a home of the user (100);
if the location of the mobile device (104) indicates the user (100) being at home, accessing, by the mobile device (104), a refrigerator's inventory of food items or images from a refrigerator's camera and identifying a plurality of carb-related food items therein;
displaying, on the mobile device (104) in response to the location of the mobile device (104) being at a home of the user (100), the plurality of carb-related food items in the refrigerator or purchased within a predefined period of time, wherein the plurality of carb-related food items are displayed in order of glycemic index;
if the location of the mobile device (104) determines the user (100) being outside of the user's home, searching for locations of carb-related food items within a predefined distance of the user's current location and accessing, by the mobile device (104), an external location service to access establishment details about establishments at the locations of carb-related food items within a predefined distance of the user's current location; and
displaying, on the mobile device (104) in response to the location of the mobile device (104) being outside of the home of the user (100), the locations of a predefined source of carbohydrates outside of the home that are within the predefined distance of the location of the mobile device (104), wherein the locations of the predefined source of carbohydrates are displayed in order of travel time proximity from the location of the mobile device (104).

2. The method of claim 1, wherein the location of the mobile device (104) is detected via global positioning system (GPS) signals, Wi-Fi signals, Bluetooth beacon signals, near-field communication (NFC) signals, or another RF communication signal.

3. The method of claim 1, wherein the location of the mobile device (104) comprises a floor of a building on which the mobile device (104) is located, and wherein the locations of the predefined source of carbohydrates outside of the home comprise a location of a vending machine.

4. The method of claim 1, wherein the location of the mobile device (104) comprises a latitude and longitude of the mobile device (104), a street address, or a user-friendly location name.

5. The method of claim 4, wherein each of the locations of the predefined source of carbohydrates are displayed with at least one of an establishment name, a latitude and longitude, an indoor identifier, an outdoor identifier, a user-friendly location name, a picture, an indication of public accessibility, or a map.

6. The method of claim 1, further comprising:
receiving a UPC code associated with at least one of the plurality of carb-related food items that indicates the glycemic index of the at least one of the plurality of carb-related food items.

7. The method of claim 1, further comprising:
receiving, from a refrigerator comprising a camera, images of the carb-related food items in the refrigerator;
comparing the images of the carb-related food items in the refrigerator with other images of carb-related food items; and
wherein the displaying of the plurality of carb-related food items comprises:
displaying the images of the carb-related food items in the refrigerator in response to a threshold level of similar features being detected between the images of the carb-related food items in the refrigerator and the other images of carb-related food items.

8. The method of claim 1, further comprising:
receiving at least one image of the carb-related food items in the refrigerator, wherein the receipt of the at least one image of the carb-related food items in the refrigerator indicates the detection of the carb-related food items by a camera of the refrigerator.

9. The method of claim 1, wherein the carb-related food items in the refrigerator are displayed on the mobile device (104) with at least one of a name, a photo, or a location in the refrigerator, and wherein the carb-related food items purchased within the predefined period of time are displayed on the mobile device (104) with at least one of a name or a photo.

10. The method of claim 1, further comprising:
receiving hours of operation for the locations of the predefined source of carbohydrates outside the home; and
displaying a subset of the locations of the predefined source of carbohydrates outside the home that are currently opened based on the hours of operation.

11. The method of claim 1, further comprising:
receiving an accepted form of payment at the locations of the predefined source of carbohydrates outside the home; and
displaying a subset of the locations of the predefined source of carbohydrates outside the home that accept at least one predefined form of payment.

12. The method of claim 1, further comprising:
receiving an indication of the locations of the predefined source of carbohydrates outside the home that sell non-diet beverages; and
displaying a subset of the locations of the predefined source of carbohydrates outside the home that sell the non-diet beverages.

13. The method of claim 1, further comprising:
receiving an indication of a user selection of a mode of transportation, wherein the locations of the predefined source of carbohydrates outside of the home are displayed in the order of travel time proximity based on the mode of transportation.

14. The method of claim 1, further comprising:
displaying the locations of the predefined source of carbohydrates outside of the home in response to the carb-related food items in the refrigerator or purchased within the predefined period of time being below a predefined glycemic index.

15. The method of claim 1, further comprising:
receiving an indication of a user selection of one of the locations of the predefined source of carbohydrates; and
providing the selected one of the locations of the predefined source of carbohydrates to a map application on the mobile device (104) for guiding the user (100) from the location of the mobile device (104) to the selected one of the locations.

## Patentansprüche

1. Verfahren, umfassend:
Erfassen eines hypoglykämischen Ereignisses, das einem Benutzer (100) zugeordnet ist, über Informationen, die von einer Blutzuckerüberwachungsvorrichtung (600) gesammelt werden;
Erfassen eines Standorts einer mobilen Vorrichtung (104), die dem Benutzer (100) zugeordnet ist, während des hypoglykämischen Ereignisses;
Bestimmen durch die mobile Vorrichtung (104), ob sich der Benutzer (100) derzeit in einem Zuhause des Benutzers (100) befindet;
Zugreifen auf das Inventar von Lebensmitteln eines Kühlschranks oder Bilder von einer Kühlschrankkamera durch die mobile Vorrichtung (104) und Identifizieren einer Vielzahl von kohlenhydrathaltigen Lebensmitteln darin, wenn der Standort der mobilen Vorrichtung (104) angibt, dass sich der Benutzer (100) zu Hause befindet;
Anzeigen der Vielzahl von kohlenhydrathaltigen Lebensmitteln, die sich in dem Kühlschrank befinden oder innerhalb eines vordefinierten Zeitraums gekauft wurden, auf der mobilen Vorrichtung (104) als Reaktion darauf, dass sich der Standort der mobilen Vorrichtung (104) in einem Zuhause des Benutzers (100) befindet, wobei die Vielzahl von kohlenhydrathaltigen Lebensmitteln in der Reihenfolge des glykämischen Index angezeigt wird;
Suchen nach Standorten von kohlenhydrathaltigen Lebensmitteln innerhalb einer vordefinierten Entfernung von dem aktuellen Standort des Benutzers und Zugreifen auf einen externen Standortdienst durch die mobile Vorrichtung (104), um auf Einrichtungsdetails über Einrichtungen an den Standorten von kohlenhydrathaltigen Lebensmitteln innerhalb einer vordefinierten Entfernung von dem aktuellen Standort des Benutzers zuzugreifen, wenn der Standort der mobilen Vorrichtung (104) bestimmt, dass sich der Benutzer (100) außerhalb des Zuhauses des Benutzers befindet; und
Anzeigen der Standorte einer vordefinierten Kohlenhydratquelle außerhalb des Zuhauses, die sich innerhalb der vordefinierten Entfernung von dem Standort der mobilen Vorrichtung (104) befinden, auf der mobilen Vorrichtung (104) als Reaktion darauf, dass sich der Standort der mobilen Vorrichtung (104) außerhalb des Zuhauses des Benutzers (100) befindet, wobei die Standorte der vordefinierten Kohlenhydratquelle in der Reihenfolge der Reisezeitnähe zu dem Standort der mobilen Vorrichtung (104) angezeigt werden.

2. Verfahren nach Anspruch 1, wobei der Standort der mobilen Vorrichtung (104) über Signale des globalen Positionsbestimmungssystems (GPS), Wi-Fi-Signale, Bluetooth-Bakensignale, Signale der Nahfeldkommunikation (NFC) oder ein anderes HF-Kommunikationssignal erfasst wird.

3. Verfahren nach Anspruch 1, wobei der Standort der mobilen Vorrichtung (104) ein Stockwerk eines Gebäudes umfasst, auf dem sich die mobile Vorrichtung (104) befindet, und wobei die Standorte der vordefinierten Kohlenhydratquelle außerhalb des Zuhauses einen Standort eines Verkaufsautomaten umfassen.

4. Verfahren nach Anspruch 1, wobei der Standort der mobilen Vorrichtung (104) einen Breitengrad und Längengrad der mobilen Vorrichtung (104), eine Straßenadresse oder einen benutzerfreundlichen Standortnamen umfasst.

5. Verfahren nach Anspruch 4, wobei jeder der Standorte der vordefinierten Kohlenhydratquelle mit mindestens einem von einem Einrichtungsnamen, einem Breitengrad und Längengrad, einer Innenraumkennung, einer Außenkennung, einem benutzerfreundlichen Standortnamen, einem Bild, einer Angabe der öffentlichen Zugänglichkeit oder einer Karte angezeigt wird.

6. Verfahren nach Anspruch 1, ferner umfassend:
Empfangen eines UPC-Codes, der mindestens einem der Vielzahl von kohlenhydrathaltigen Lebensmitteln zugeordnet ist, der den glykämischen Index des mindestens einen der Vielzahl von kohlenhydrathaltigen Lebensmitteln angibt.

7. Verfahren nach Anspruch 1, ferner umfassend:
Empfangen von Bildern der kohlenhydrathaltigen Lebensmittel in dem Kühlschrank von einem Kühlschrank, der eine Kamera umfasst;
Vergleichen der Bilder der kohlenhydrathaltigen Lebensmittel in dem Kühlschrank mit anderen Bildern von kohlenhydrathaltigen Lebensmitteln; und
wobei das Anzeigen der Vielzahl von kohlenhydrathaltigen Lebensmitteln Folgendes umfasst:
Anzeigen der Bilder der kohlenhydrathaltigen Lebensmittel in dem Kühlschrank als Reaktion darauf, dass ein Schwellenwert ähnlicher Merkmale zwischen den Bildern der kohlenhydrathaltigen Lebensmittel in dem Kühlschrank und den anderen Bildern von kohlenhydrathaltigen Lebensmitteln erfasst wird.

8. Verfahren nach Anspruch 1, ferner umfassend:
Empfangen mindestens eines Bildes der kohlenhydrathaltigen Lebensmittel in dem Kühlschrank, wobei der Empfang des mindestens einen Bildes der kohlenhydrathaltigen Lebensmittel in dem Kühlschrank die Erfassung der kohlenhydrathaltigen Lebensmittel durch eine Kamera des Kühlschranks angibt.

9. Verfahren nach Anspruch 1, wobei die kohlenhydrathaltigen Lebensmittel in dem Kühlschrank auf der mobilen Vorrichtung (104) mit mindestens einem von einem Namen, einem Foto oder einem Standort in dem Kühlschrank angezeigt werden, und wobei die kohlenhydrathaltigen Lebensmittel, die innerhalb des vordefinierten Zeitraums gekauft wurden, auf der mobilen Vorrichtung (104) mit mindestens einem von einem Namen oder einem Foto angezeigt werden.

10. Verfahren nach Anspruch 1, ferner umfassend:
Empfangen von Betriebsstunden für die Standorte der vordefinierten Kohlenhydratquelle außerhalb des Zuhauses; und
Anzeigen einer Teilmenge der Standorte der vordefinierten Kohlenhydratquelle außerhalb des Zuhauses, die aktuell geöffnet sind, basierend auf den Betriebsstunden.

11. Verfahren nach Anspruch 1, ferner umfassend:
Empfangen einer akzeptierten Zahlungsform an den Standorten der vordefinierten Kohlenhydratquelle außerhalb des Zuhauses; und
Anzeigen einer Teilmenge der Standorte der vordefinierten Kohlenhydratquelle außerhalb des Zuhauses, die mindestens eine vordefinierte Zahlungsform akzeptieren.

12. Verfahren nach Anspruch 1, ferner umfassend:
Empfangen einer Angabe der Standorte der vordefinierten Kohlenhydratquelle außerhalb des Zuhauses, die kalorienhaltige Getränke verkaufen; und
Anzeigen einer Teilmenge der Standorte der vordefinierten Kohlenhydratquelle außerhalb des Zuhauses, die die kalorienhaltige Getränke verkaufen.

13. Verfahren nach Anspruch 1, ferner umfassend:
Empfangen einer Angabe einer Benutzerauswahl eines Transportmodus, wobei die Standorte der vordefinierten Kohlenhydratquelle außerhalb des Zuhauses in der Reihenfolge der Reisezeitnähe basierend auf dem Transportmodus angezeigt werden.

14. Verfahren nach Anspruch 1, ferner umfassend:
Anzeigen der Standorte der vordefinierten Kohlenhydratquelle außerhalb des Zuhauses als Reaktion darauf, dass die kohlenhydrathaltigen Lebensmittel, die sich in dem Kühlschrank befinden oder innerhalb des vordefinierten Zeitraums gekauft wurden, unter einem vordefinierten glykämischen Index liegen.

15. Verfahren nach Anspruch 1, ferner umfassend:
Empfangen einer Angabe einer Benutzerauswahl eines der Standorte der vordefinierten Kohlenhydratquelle; und
Bereitstellen des ausgewählten der Standorte der vordefinierten Kohlenhydratquelle für eine Kartenanwendung auf der mobilen Vorrichtung (104), um den Benutzer (100) von dem Standort der mobilen Vorrichtung (104) zu dem ausgewählten der Standorte zu führen.

## Revendications

1. Procédé comprenant :
la détection, par le biais d'informations rassemblées à partir d'un dispositif de surveillance de glycémie (600), d'un événement hypoglycémique associé à un utilisateur (100) ;
la détection d'un emplacement d'un dispositif mobile (104) associé à l'utilisateur (100) pendant l'événement hypoglycémique ;
la détermination, par le dispositif mobile (104), si l'utilisateur (100) est actuellement au domicile de l'utilisateur (100) ;
si l'emplacement du dispositif mobile (104) indique que l'utilisateur (100) est à son domicile, l'accès, par le dispositif mobile (104), à un inventaire du réfrigérateur de produits alimentaires ou à des images provenant d'une caméra du réfrigérateur et l'identification d'une pluralité de produits alimentaires contenant des glucides à l'intérieur de celui-ci ;
l'affichage, sur le dispositif mobile (104) en réponse au fait que l'emplacement du dispositif mobile (104) est au domicile de l'utilisateur (100), de la pluralité de produits alimentaires contenant des glucides dans le réfrigérateur ou achetés pendant une période de temps prédéfinie, la pluralité de produits alimentaires contenant des glucides étant affichés par ordre d'indice glycémique ;
si l'emplacement du dispositif mobile (104) détermine que l'utilisateur (100) est à l'extérieur du domicile de l'utilisateur, la recherche d'emplacements de produits alimentaires contenant des glucides à moins d'une distance prédéfinie de l'emplacement actuel de l'utilisateur et l'accès, par le dispositif mobile (104), à un service d'emplacements externes pour accéder à des détails d'établissement au sujet d'établissements aux emplacements des produits alimentaires contenant des glucides à moins d'une distance prédéfinie de l'emplacement actuel de l'utilisateur ; et
l'affichage, sur le dispositif mobile (104) en réponse au fait que l'emplacement du dispositif mobile (104) est à l'extérieur du domicile de l'utilisateur (100), des emplacements d'une source prédéfinie de glucides à l'extérieur du domicile qui sont à moins de la distance prédéfinie de l'emplacement du dispositif mobile (104), les emplacements de la source prédéfinie de glucides étant affichés par ordre de proximité en temps de trajet par rapport à l'emplacement du dispositif mobile (104).

2. Procédé selon la revendication 1, dans lequel l'emplacement du dispositif mobile (104) est détecté par le biais de signaux du système de positionnement global (GPS), de signaux Wi-Fi, de signaux de balise Bluetooth, de signaux de communication en champ proche (NFC) ou d'un autre signal de communication RF.

3. Procédé selon la revendication 1, dans lequel l'emplacement du dispositif mobile (104) comprend un sol d'un bâtiment sur lequel le dispositif mobile (104) est situé, et dans lequel les emplacements de la source prédéfinie de glucides à l'extérieur du domicile comprennent un emplacement d'un distributeur automatique.

4. Procédé selon la revendication 1, dans lequel l'emplacement du dispositif mobile (104) comprend une latitude et une longitude du dispositif mobile (104), une adresse postale ou un nom d'emplacement facile à comprendre.

5. Procédé selon la revendication 4, dans lequel chacun des emplacements de la source prédéfinie de glucides est affiché avec au moins un parmi un nom d'établissement, une latitude et une longitude, un identifiant intérieur, un identifiant extérieur, un nom d'emplacement facile à comprendre, une image, une indication d'accessibilité pour le public ou une carte.

6. Procédé selon la revendication 1, comprenant en outre :
la réception d'un code UPC associé à au moins l'un de la pluralité de produits alimentaires contenant des glucides qui indique l'indice glycémique de l'au moins un de la pluralité de produits alimentaires contenant des glucides.

7. Procédé selon la revendication 1, comprenant en outre :
la réception, à partir d'un réfrigérateur comprenant une caméra, d'images des produits alimentaires contenant des glucides dans le réfrigérateur ;
la comparaison des images des produits alimentaires contenant des glucides dans le réfrigérateur à d'autres images de produits alimentaires contenant des glucides ; et
dans lequel l'affichage de la pluralité de produits alimentaires contenant des glucides comprend :
l'affichage des images de produits alimentaires contenant des glucides dans le réfrigérateur en réponse à la détection d'un niveau seuil de caractéristiques similaires entre les images des produits alimentaires contenant des glucides dans le réfrigérateur et les autres images de produits alimentaires contenant des glucides.

8. Procédé selon la revendication 1, comprenant en outre :
la réception d'au moins une image des produits alimentaires contenant des glucides dans le réfrigérateur, la réception de l'au moins une image des produits alimentaires contenant des glucides dans le réfrigérateur indiquant la détection des produits alimentaires contenant des glucides par une caméra du réfrigérateur.

9. Procédé selon la revendication 1, dans lequel les produits alimentaires contenant des glucides dans le réfrigérateur sont affichés sur le dispositif mobile (104) avec au moins un parmi un nom, une photo ou un emplacement dans le réfrigérateur, et dans lequel les produits alimentaires contenant des glucides achetés pendant la période de temps prédéfinie sont affichés sur le dispositif mobile (104) avec au moins un parmi un nom ou une photo.

10. Procédé selon la revendication 1, comprenant en outre :
la réception des heures d'ouverture pour les emplacements de la source prédéfinie de glucides à l'extérieur du domicile ; et
l'affichage d'un sous-ensemble des emplacements de la source prédéfinie de glucides à l'extérieur du domicile qui sont actuellement ouverts en se basant sur les heures d'ouverture.

11. Procédé selon la revendication 1, comprenant en outre :
la réception d'un mode de paiement accepté au niveau des emplacements de la source prédéfinie de glucides à l'extérieur du domicile ; et
l'affichage d'un sous-ensemble des emplacements de la source prédéfinie de glucides à l'extérieur du domicile qui acceptent au moins un mode de paiement prédéfini.

12. Procédé selon la revendication 1, comprenant en outre :
la réception d'une indication des emplacements de la source prédéfinie de glucides à l'extérieur du domicile qui vendent des boissons non allégées en sucre ; et
l'affichage d'un sous-ensemble des emplacements de la source prédéfinie de glucides à l'extérieur du domicile qui vendent les boissons non allégées en sucre.

13. Procédé selon la revendication 1, comprenant en outre :
la réception d'une indication d'une sélection par l'utilisateur d'un mode de transport, dans lequel les emplacements de la source prédéfinie de glucides à l'extérieur du domicile sont affichés par ordre de proximité en temps de trajet en se basant sur le mode de transport.

14. Procédé selon la revendication 1, comprenant en outre :
l'affichage des emplacements de la source prédéfinie de glucides à l'extérieur du domicile en réponse au fait que les produits alimentaires contenant des glucides dans le réfrigérateur ou achetés pendant la période de temps prédéfinie sont en dessous d'un indice glycémique prédéfini.

15. Procédé selon la revendication 1, comprenant en outre :
la réception d'une indication d'une sélection par l'utilisateur de l'un des emplacements de la source prédéfinie de glucides ; et
la mise à disposition de l'emplacement sélectionné parmi les emplacements de la source prédéfinie de glucides dans une application cartographique sur le dispositif mobile (104) pour guider l'utilisateur (100) de l'emplacement du dispositif mobile (104) à l'emplacement sélectionné parmi les emplacements.
